# EUROPÄISCHE PATENTSCHRIFT

(11) **EP 1 075 292 B1**
(45) Veröffentlichungstag und Bekanntmachung des Hinweises auf die Patenterteilung: **14.08.2002**
(21) Anmeldenummer: 99929032.3
(22) Anmeldetag: 24.04.1999
(51) Int. Cl.: A61M 5/20

(54) **INJEKTIONSVORRICHTUNG**
INJECTION DEVICE
DISPOSITIF D'INJECTION

(30) Priorität: 30.04.1998 DE 19819409
(43) Veröffentlichungstag der Anmeldung: 14.02.2001
(73) Patentinhaber: SCHERING AKTIENGESELLSCHAFT, 13342 Berlin (DE)
(72) Erfinder: WEBER, Wilfried, D-72296 Schopfloch (DE)
(74) Vertreter: Frank, Gerhard, Dipl.-Phys.
(86) Internationale Anmeldenummer: DE9901244
(87) Internationale Veröffentlichungsnummer: WO9956805

(56) Entgegenhaltungen:
- DE-A- 3 113 977
- DE-U- 8 912 091
- FR-A- 2 567 760
- FR-A- 2 733 155
- US-A- 5 026 349
- US-A- 5 137 516

## Beschreibung

### Technisches Gebiet der Erfindung

Die Erfindung betrifft eine Injektionsvorrichtung mit einer Spritze, insbesondere einer Einmal-Spritze, zur Injektion einer Injektionsflüssigkeit unter die Haut.

Die Durchführung von medizinisch sachgemäßen Injektionen mittels einer Spritze setzt ein Mindestmaß an medizinischem Wissen und praktischem Geschick voraus. Die Einhaltung dieser Anforderungen ist im stationären Krankenhaus- oder Pflegebereich in der Regel gewährleistet, da die Handhabung von Spritzen hier von medizinisch geschultem Fachpersonal durchgeführt wird; mit der Zunahme von chronischen Krankheiten infolge der zunehmenden Lebenserwartung der Bevölkerung nimmt jedoch auch der häusliche Pflegebereich zu.

Dies hat zur Folge, daß die Handhabung von Spritzen zunehmend von medizinischen Laien als Patienten durchführbar sein muß, zur Selbstbehandlung zu Hause; abgesehen von einer gewissen Hemmschwelle beim Ansetzen einer Spritze ist die Einhaltung der oben genannten Kriterien auch mit zunehmendem Alter der Patienten nicht mehr gewährleistet, beispielsweise durch motorische Störungen oder Sehbeeinträchtigungen.

Dies hat in den letzten Jahrzehnten zunehmend zur Konzeption von teil- oder vollautomatisch arbeitenden Injektionsvorrichtungen geführt, in die insbesondere übliche Fertigspritzen eingelegt werden können, so daß dem Patienten nur noch verbleibt, das Vorderende dieser Injektionsvorrichtung auf der Injektionsstelle zu positionieren und dann eine Auslöseeinrichtung in der Injektionsvorrichtung insbesondere durch Fingerdruck zu betätigen, worauf das Einstechen der Nadel und die Injektion der Injektionsflüssigkeit dann meistens unter Einwirkung einer Federeinrichtung durchgeführt wird.Beispiele für solche Injektionsvorrichtungen zeigen die DE 29 50 140. C2, die DE 14 91 842 C2, die DE 24 36 000 C2, die EP 0 144 625 B1, die EP 0 516 473 B1 und die EP 0 577 448 A1.

### Stand der Technik

Die Erfindung geht aus von einer gattungsgemäßen Vorrichtung, wie sie durch die FR 2 733 155 A beschrieben wird. Diese Injektionsvorrichtung beinhaltet in einem Gehäuse gehaltene Einbauteile, die unter der Wirkung einer Feder als Antriebsmittel die Spritze derart beaufschlagen, daß durch eine sukzessive Linearverschiebung der Spritze und der Injektionsnadel im Gehäuse zunächst die Einführung der Injektionsnadel der Spritze unter die Haut und danach erst die Injektion der Injektionsflüssigkeit erfolgt. Nach erfolgter Injektion werden dann diese Bauteile durch eine Gegenbewegung wieder in ihre Ausgangsposition zurückgeführt. Innerhalb dieses Bewegungsablaufs ist es erforderlich, die entleerte Einmal-Spritze aus der Injektionsvorrichtung zu entnehmen und durch eine gefüllte, neue Einmal-Spritze zu ersetzen, damit dann der Injektionsvorgang wieder neu gestartet werden kann. Hierzu weist zu diesem Zweck das Gehäuse eine axial verschiebbare Steuerhülse auf, die nach Rückführung der die Linearverschiebung bewirkenden Einbauteile in eine Öffnungsposition gezogen wird, worauf eine neue Einwegspritze eingelegt werden kann und die Steuerhülse in eine Schließ- und Funktionsposition geschoben wird. Die Spannung der den Injektionshub bewirkenden Feder wird hierbei beim Zurückziehen der Steuerhülse erzeugt, bis eine Arretierposition erreicht ist,wozu die beim Zusammendrücken zu überwindende Federkraft in voller Höhe als Zug- oder Druckkraft vom Patienten aufgebracht werden muß.Der Durchlauf eines kompletten Injektionszyklus erfordert bei dieser Konzeption eine Mehrzahl aufeinanderfolgender Handgriffe in festgelegter Reihenfolge.
Die US 5,137,516 A zeigt eine Betätigungseinheit und eine Gehäuseeinheit zur Aufnahme der Spritze, die zur Betätigung der Spritze axial fluchtend aufeinandergesetzt und miteinander verbunden werden müssen. Hierzu ist eine Schraubhülse vorgesehen, die Bestandteil der Gehäuseeinheit ist, und auf die die Betätigungseinheit nach Einlegen einer gefüllten Spritze aufgeschraubt werden kann und von der nach erfolgter Injektion die Betätigungseinheit wieder abgeschraubt werden muss, um die entleerte Spritze zu entnehmen.
Die DE 89 12 091 U zeigt eine einstufig arbeitende Injektionsvorrichtung, bei der Einstechen und Injektion gleichzeitig ablaufen. Wie bei der oben genannten US 5,137,516 sind eine Betätigungseinheit und eine Gehäuseeinheit zur Aufnahme der Spritze vorgesehen, die hier allerdings fest parallel zueinander zugeordnet sind und so eine Baueinheit bilden. In der Betätigungseinheit ist eine "Säule" axial geführt, die an ihrem oberen Ende eine Platte aufweist, die durch Verdrehung der Säule die Einführöffnung der Gehäuseeinheit sperrt oder freigibt, und dadurch eine Schließ- und Funktionsposition und eine Öffnungsposition bezüglich der Gehäuseeinheit definiert.
Die Handbabung dieser beiden letztgenannten Injektionsvorrichtungen beim Wechsel der Einmalspritze erfordert insbesondere beim paß- und richtungsgenauen Zusammenführen der beiden Bestandteile mit wieder eingelegter, neuer Fertigspritze mehrere Handhabungsgriffe und somit einiges manuelles Geschick, das bei dem oben angesprochenen Personenkreis chronisch kranker, älterer Menschen nicht vorausgesetzt werden kann; insbesondere für diesen Personenkreis sind diese vorbekannten Lösungen daher wenig geeignet.

### Gegenstand der Erfindung

Die wesentliche Aufgabe der Erfindung besteht darin,die gattungsgemäße Injektionsvorrichtung so weiterzubilden, daß bei einfachem mechanischem Aufbau die zur Handhabung erforderlichen Handgriffe seitens des Patienten auf ein Minimum reduziert werden, insbesondere Entnahme und Einlegen der Einmalspritze so vereinfacht wird, daß nur minimale Anforderungen an die physischen und psychischen (Rest-) Fähigkeiten des Patienten gestellt werden.

Erfindungsgemäß wird diese Aufgabe gemäß dem kennzeichnenden Teil des Patentanspruchs 1 gelöst.

Der wesentliche Gedanke der erfindungsgemäßen Lösung besteht darin, in einem zentralen Bauteil, der Steuerhülse, die manuell extrem leicht zu handhaben ist, da sie lediglich eine Verdrehung gegenüber dem Gehäuse erfordert, eine Vielzahl von Antriebs-, Steuerungs- und Sicherheitsfunktionen zu vereinigen:

Die Steuerhülse ist insofern Teil des Gehäuses, als daß sie anstelle beispielsweise einer Klappe zum Verschluß und zur Freigabe des Innenraums des Gehäuses dient, in dem die Fertigspritze gehalten ist.

Die Steuerhülse ist Teil der Antriebsmittel insoweit, als daß bei der Öffnung des Gehäuses durch Verdrehung der Steuerhülse ein Gegenhub von Schlitten und Stößel gegen die Wirkung einer die Injektionshübe ausführenden Feder erzeugt wird, der diese beiden Bauteile wieder in ihre ursprüngliche Arretierposition befördert, separate Maßnahmen und Handgriffe sind für diesen zwingend notwendigen Verfahrensablauf nicht erforderlich.

Die Steuerhülse ist insofern auch ein Steuerungsmittel, als sie abhängig von ihrer Position die Auslöseeinrichtung der Injektionsvorrichtung sperrt oder deren Aktivierung zuläßt.

Die Steuerhülse ermöglicht auch weitere vorteilhafte Ausgestaltungen der erfindungsgemäßen Lösung zur Erhöhung der Betriebssicherheit und des Bedienungskomforts insofern, als sie unmittelbar eine Auswurfeinrichtung betätigt, die nach erfolgter Injektion automatisch beim Öffnen des Gehäuses mittels der Steuerhülse die leere Fertigspritze dem Patienten entgegendrückt und somit die Entnahme erleichtert und sie wird auch ausgenutzt zu einer Rückmeldung der Position und des Betriebszustandes der Injektionsvorrichtung, die für den Patienten sensorisch leicht wahrnehmbar ist.

Weitere erfindungsgemäße vorteilhafte Ausgestaltungen betreffen eine deutlich wahrnehmbare, akustische Signaleinrichtung, mit der das Ende eines Injektionsvorganges dem Patienten angezeigt wird.

Diese Vielfalt von Funktionen läßt sich mit einem einfachen Aufbau der Injektionsvorrichtung realisieren; so sind die wesentlichen Bauteile der Injektionsvorrichtung beispielsweise durch Spritzgußteile realisierbar, die leicht montierbar sind, so daß die Injektionsvorrichtung von Material- und Arbeitsaufwand kostengünstig herstellbar ist.

### Kurze Beschreibung der Zeichnungen

Bevorzugte Ausführungsbeispiele der erfindungsgemäßen Injektionseinrichtung werden nun anhand von Zeichnungen näher erläutert. Es zeigen:
- Fig. 1: einen ersten Längsschnitt durch die Injektionsvorrichtung in ihrer Öffnungsposition zum Einlegen oder Entnehmen der Spritze in der Ebene I-I der Fig. 3,
- Fig. 2: einen ersten Querschnitt durch die Injektionsvorrichtung in ihrer Öffnungsposition in der Schnittebene A-A der Fig. 1,
- Fig. 3: einen zweiten Längsschnitt durch die Injektionsvorrichtung in ihrer Öffnungsposition in der Schnittebene III-III der Fig. 1,
- Fig. 4: eine erste Seitenansicht der Signaleinrichtungen der Injektionsvorrichtung in der Schnittebene der Fig. 1,
- Fig. 5: eine zweite Seitenansicht der Signaleinrichtungen der Injektionsvorrichtung in der Schnittebene der Fig. 3,
- Fig. 6: einen dritten Längsschnitt durch die Injektionsvorrichtung unmittelbar am Beginn des zweiten Hubes, in der Ebene der Fig. 1,
- Fig. 7: einen vierten Längsschnitt durch die Injektionsvorrichtung während des zweiten Hubes, in der Ebene der Fig. 1,
- Fig. 8: einen fünften Längsschnitt durch die Injektionsvorrichtung am Endes des zweiten Hubes, in der Ebene der Fig. 1,
- Fig. 9: einen zweiten Querschnitt durch die Injektionsvorrichtung am Ende des zweiten Hubes, in der Ebene B-B der Fig. 8,
- Fig. 10: einen sechsten Längsschnitt durch die Injektionsvorrichtung am Ende des zweiten Hubes, in der Ebene X-X der Fig. 8,
- Fig. 11: eine perspektivische Darstellung des Mantels MI der Steuerhülse mit einer Steuerkurve,
- Fig. 12: eine Abwicklung der inneren Mantelfläche MI der Figur 11 mit einer Steuerkurve,
- Figur 13: eine Abwicklung der inneren Mantelfläche der Steuerhülse gemäß einer Variante mit zwei Steuerkurven,
- Figur 14A,B: Schnitte in den Ebenen C-C und D-D durch den Mantelteil der Steuerhülse gemäß Figur 13,
- Figur 15A,B,C: Schnitte und perspektivische Darstellungen der Auswurfeinrichtung,
- Figur 16A,B,C, D: perspektivische Darstellungen eines Ausführungsbei spiels des Schlittens mit integriertem Getriebe,
- Figur 17A,B,C: Ansichten und Längsschnitt durch den Getriebeschlitten der Figur 16,
- Figur 18: die Injektionsvorrichtung mit eingebautem Getriebeschlitten, und
- Figur 19: einen Horizontal-Teilschnitt durch die Injektionsvorrichtung im Bereich des Getriebeschlittens.

### Beschreibung des bevorzugten Ausführungsbeispiels

Zunächst soll der grundsätzliche Aufbau der erfindungsgemäßen Injektionsvorrichtung erläutert werden, danach dann die einzelnen Funktionsabschnitte innerhalb eines Arbeitszyklus.

Die Injektionsvorrichtung besitzt als sozusagen "mechanisches Traggerüst" ein Gehäuse 7, dessen Vorderabschnitt 7C dort, wo die Nadel 13 der Spritze 1 austritt, konisch zuläuft, in seinem Mittelabschnitt 7B im wesentlichen zylindrisch ausgeführt ist und in einen rückwärtigen, kappenähnlich ausgebildeten Handhabungsabschnitt 7A mit Gehäuserückwand 74 übergeht, der eine Ringnut 75 aufweist. Die Längsachse F dieses Gehäuses 7 bildet die Längsachse, d.h., diejenige Achse, in der die Spritze 1 gehalten ist und in der folglich auch die wesentlichen Bewegungen und Steuerungsvorgänge zur Injektion ablaufen.

Das Gehäuse 7 weist in seinem an den Vorderabschnitt 7C anschließenden Mittelabschnitt 7B eine Ladeöffnung L7 auf, die sich über einen spitzen Umfangswinkel bezogen auf die Längsachse F erstreckt, im dargestellten Ausführungsbeispiel erstreckt sich der mittlere Teil des Gehäuses 7 über einen Winkel α 2 von etwa 270°, so daß die Ladeöffnung L7 des Gehäuses 7 etwa einen Umfangsbereich von 90° in Anspruch nimmt. Über diese Ladeöffnung L7 ist der Innenraum des Gehäuses 7 zum Einlegen und zur Entnahme einer Spritze 1 zugänglich.

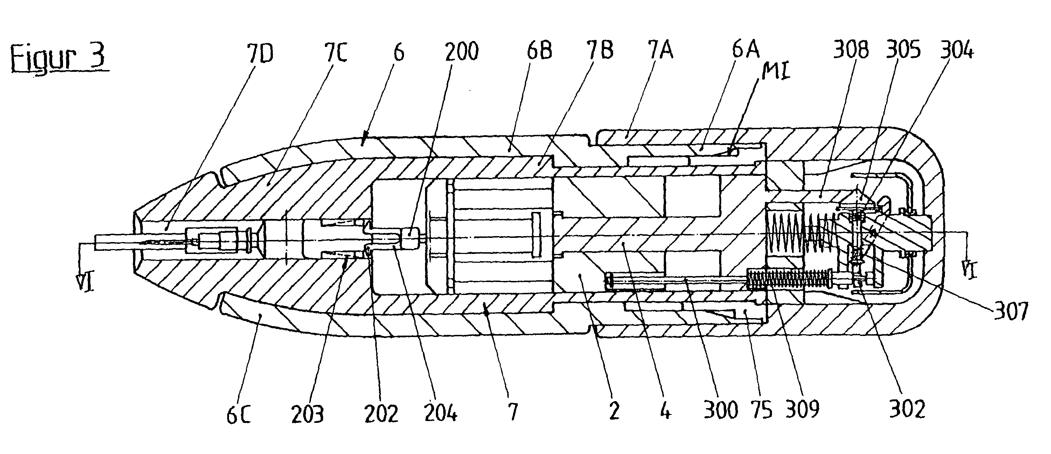

Zur Verdeutlichung des grundsätzlichen Aufbaus des Gehäuses 7 ist dieses in der Figur 3 eng schraffiert dargestellt.

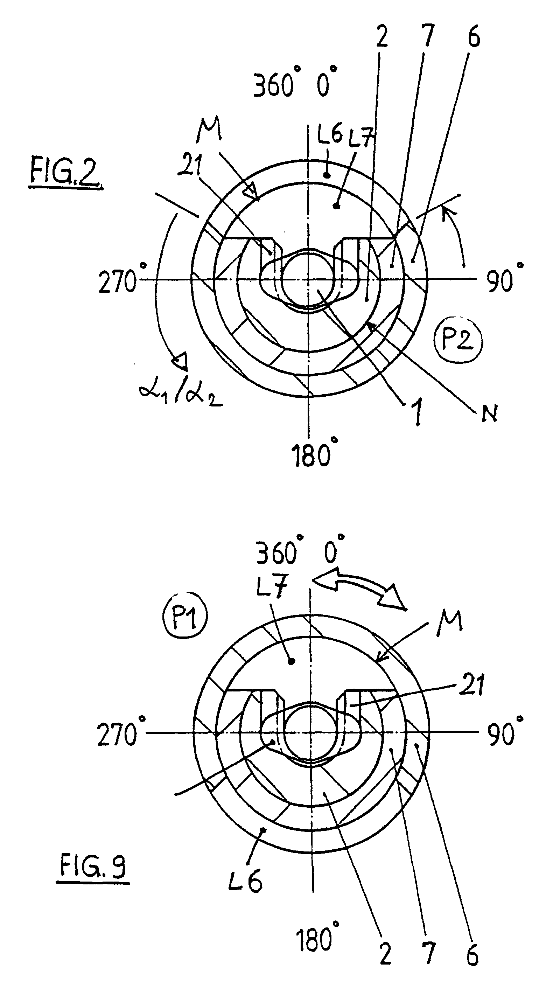

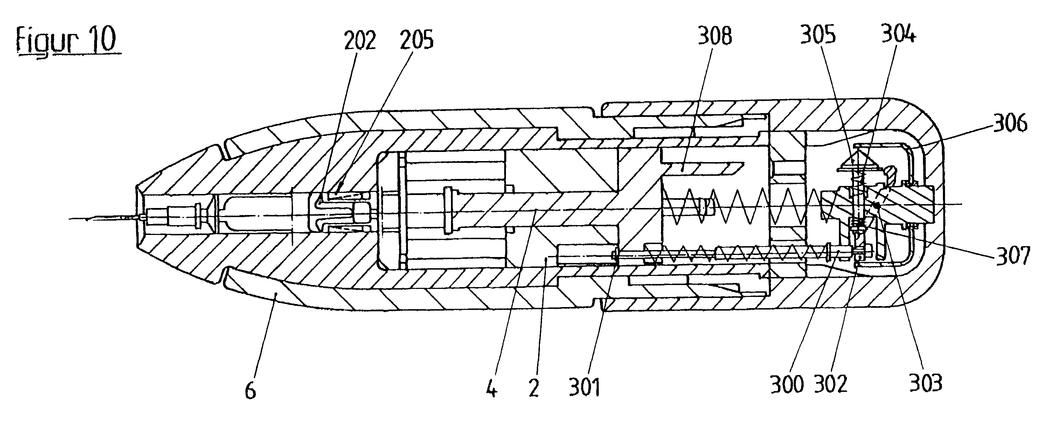

Auf und in diesem Gehäuse 7 ist als besonders erfindungswesentliches Funktionsbauteil eine Steuerhülse 6 gehalten, die koaxial zum Gehäuse 7 um dessen Längsachse F verdrehbar ist, wie dies insbesondere in den Figuren 2 und 9 dargestellt ist. Die Steuerhülse 6 weist zunächst einen vorderen, verjüngten Führungsabschnitt 6C auf, der weitgehend formschlüssig auf dem sich nach vorne verjüngenden Vorderabschnitt 7C des Gehäuses 7 aufsitzt und auf diesem Abschnitt des Gehäuses 7 drehbar gehalten ist. Daran schließt ein im wesentlichen zylindrischer Mittelabschnitt 6B an, der über einen Teil seines Umfangs eine Ladeöffnung L6 zum Einlegen und Entnehmen der Spritze 1 aus dem Gehäuse 7 aufweist. Dieser Mittelbereich erstreckt sich über einen Winkel α1 von ebenfalls etwa 270° entsprechend dem Gehäuse 7, so daß für die Ladeöffnung L6 ein Öffnungswinkel von ebenfalls etwa 90° verbleibt.

Eine erste erfindungswesentliche Funktion dieser Konzeption von Steuerhülse 6 und Gehäuse 7 besteht darin, daß durch Verdrehen der Steuerhülse 6 auf dem Gehäuse 7 die Steuerhülse 6 auf der Mantelfläche M des Gehäuses 7 gleitet, so daß in einer Öffnungs- und Sicherungsposition P2 (Figur 2) die beiden Ladeöffnungen L6 der Steuerhülse 6 einerseits und L7 des Gehäuses 7 andererseits weitgehend deckungsgleich sind, d.h. in dieser Öffnungs- und Sicherungsposition P2 gibt die Steuerhülse 6 die Ladeöffnung L7 des Gehäuses frei, so daß der Benutzer Zugriff auf die in der Längsachse F gehaltene Spritze 1 hat.

Durch Verdrehen der Steuerhülse 6 auf der Mantelfläche M (symbolisiert durch den Doppelpfeil in Figur 9), läßt sich eine Position darstellen, in der die Steuerhülse 6 die Ladeöffnung L7 des Gehäuses 7 vollständig verschließt (Figur 9) und somit eine Schließ- und Funktionsposition P1 der erfindungsgemäßen Injektionsvorrichtung definiert.

Diese "Umschaltfunktion" der Steuerhülse 6 zwischen Schließ- und Funktionsposition P1 und Öffnungs- und Sicherungsposition P2 ist eine der wesentlichen Funktionen der Steuerhülse.

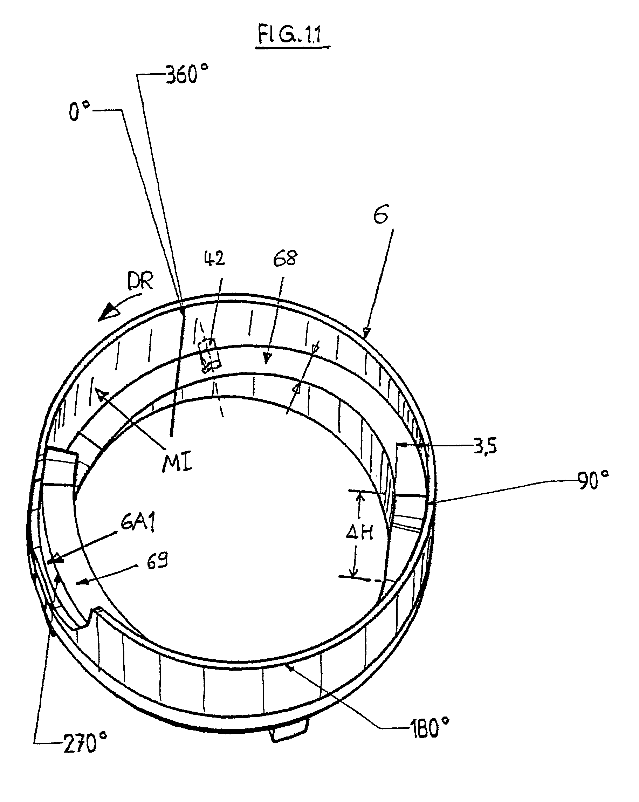

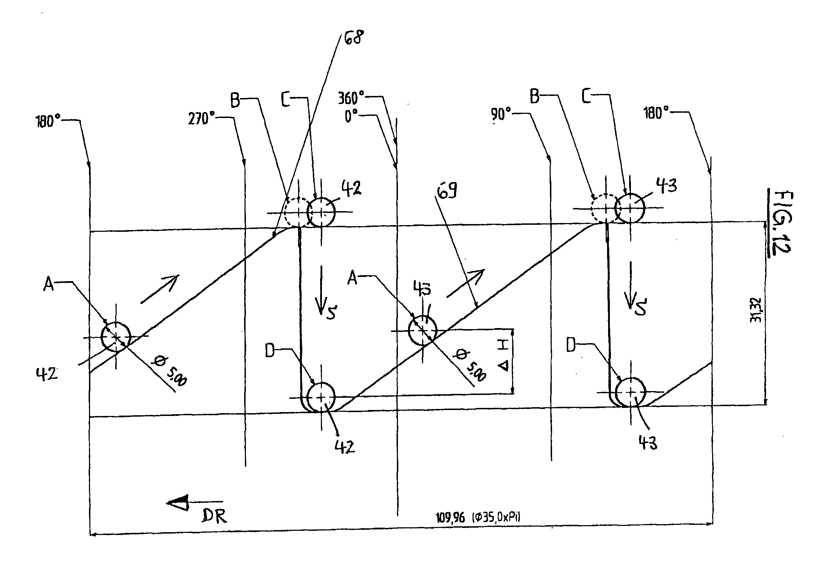

Eine andere wichtige Aufgabe, nämlich die Antriebs- und Steuerfunktion für die Vielzahl, weiter unten noch erläuterter Funktionsbauteile, die im Gehäuse angeordnet sind, besteht im Zusammenwirken des rückwärtigen, zylindrischen Steuerabschnittes 6A der Steuerhülse 6 mit einer Ringnut 75 des Gehäuses 7. Die innere Mantelfläche MI des zylindrischen Steuerabschnittes 6A der Steuerhülse 6 ist mit Profilen oder Profilbahnen versehen, die als Steuerkurven 64,65,66 und 67 ausgebildet sind, wie dies in der Abwicklung der inneren Mantelfläche MI der Figur 11 und 12 dargestellt ist. Diese Steuerkurven arbeiten mit Steuerelementen von weiteren, innerhalb des Gehäuses 7 gehaltenen Bauteilen zusammen, die zur Erzeugung von zwei Injektionshüben dienen und deren Aufbau im folgenden beschrieben wird:

Der Mittelabschnitt 7B des Gehäuses 7 (äußere Mantelfläche M) ist hohlzylindrisch ausgebildet, d.h. er weist auch eine innere Mantelfläche N auf (Figur 2). In dem dadurch geschaffenen Innenraum des Mittelabschnitts 7B gleitet ein teilweise zylindrisch ausgebildeter Schlitten 2. Dieser Schlitten 2 weist einen rückwärtigen, ersten Abschnitt 2A auf, der als Hohlzylinder ausgebildet ist, und einen ebenfalls im Gehäuse 7 geführten vorderen, etwa halbzylindrischen zweiten Abschnitt 2B, der eine nutförmige oder schlitzartige Halterung 21 zur Positionierung des Spritzenbundes 12 der Spritze 1 aufweist, so daß die Spritze 1 dadurch in Richtung der Längsachse F im Schlitten 2 ortsfest positioniert und gehalten ist, d.h. insbesondere, daß die Axialposition der Injektionsnadel 13 der Spritze 1 in der Längsachse F eindeutig durch die Position des Spritzenbundes 12 und damit die Axialposition des Schlittens 2 im Gehäuse 7 festgelegt ist.

Die Axialbewegung des Schlittens 2 in der Längsachse F ist durch geeignete Anschlag- und Sicherungsmittel innerhalb des Gehäuses 7 zwischen einer Arretierposition A1 und einer Vorschubposition V1 begrenzt, d.h., in der Vorschubposition V1 befindet sich die Spritze 1 mit ihrer Injektionsnadel 13 in der aus der Spitze des Gehäuses 7 ausgefahrenen, zur Injektion funktionsbereiten Position, in der Arretierposition A1 befindet sich der Schlitten in seiner rückwärtigen Position, in der die Injektionsnadel 13 innerhalb des Austrittskanals 7D im Vorderabschnitt 7C des Gehäuses 7 versenkt ist.

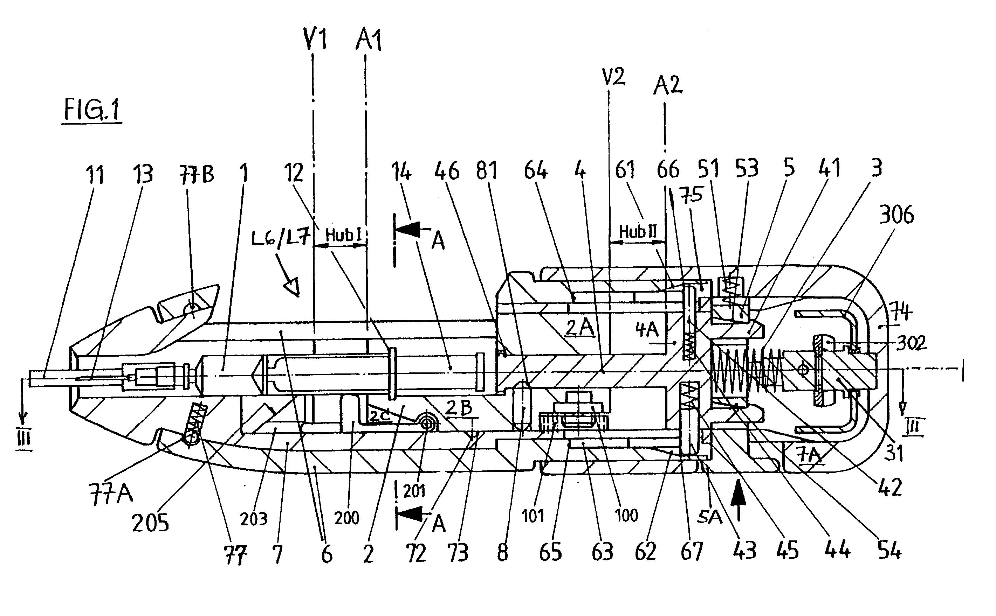

Diese Axialbewegung des Schlittens 2 zwischen Arretierposition A1 und Vorschubposition V1 wird im folgenden als "erster Hub" H1 (Hub I in Figur 1) bezeichnet. Dieser Hub dient somit zur Bewegung der Spritze 1 als ganze Funktionseinheit nach vorne, d.h. zum Einstechen der Nadel 13 in die medizinisch erforderliche Tiefe des Gewebes unter der Haut, wenn die Austrittsöffnung des Kanals 7D auf der Haut aufsitzt.

Im ersten Abschnitt 2A des Schlittens 2 ist ein stempelartig ausgebildeter Stößel 4 mittig axial verschiebbar gelagert. Dieser Stößel 4 ist an seinem zum Handhabungsabschnitt 7A des Gehäuses 7 zeigenden Ende als Führungsabschnitt 4A ausgebildet, der den gleichen Außendurchmesser aufweist wie der erste Abschnitt 2A des Schlittens 2, so daß diese beiden Bauteile im Mittelabschnitt 7B des Gehäuses 7 axial verschiebbar sind.

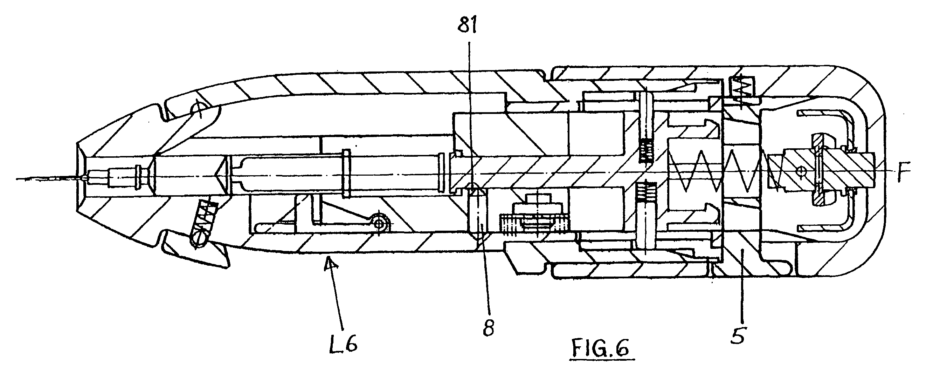

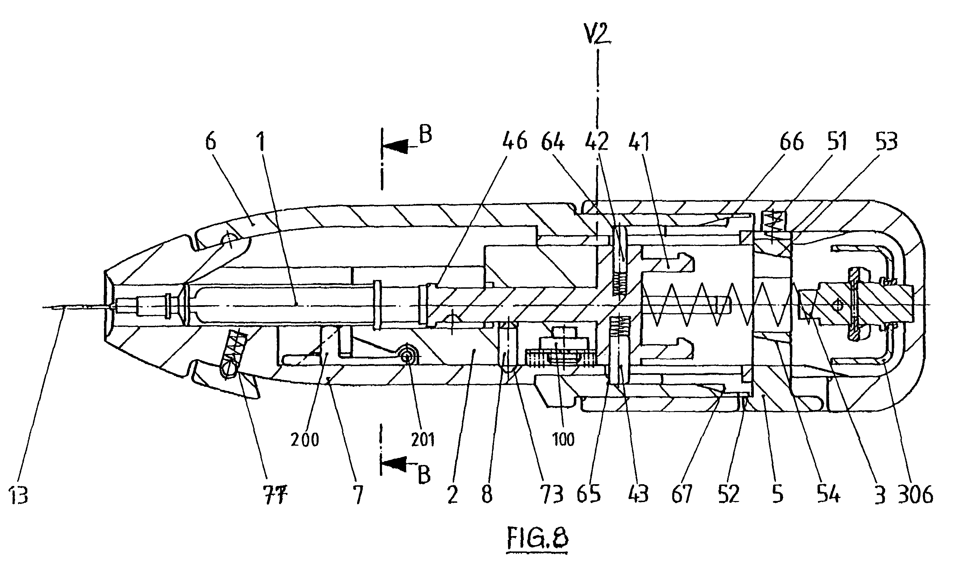

Die zum vorderen Ende der Injektionsvorrichtung zeigende Stirnseite des Stößels 4 liegt unmittelbar der Stirnseite des Spritzenkolbens 14 gegenüber, so daß eine Axialverschiebung des Stößels 4 im Schlitten 2 (in dem die Spritze 1 ortsfest gehalten ist) zu einer Beaufschlagung des Spritzenkolbens 14 und somit zum Einschieben des Spritzenkolbens 14 in die Spritze 1 und zur Abgabe der Injektionsflüssigkeit durch die Injektionsnadel 13 führt. Auch der Stößel 4 ist somit zwischen zwei Positionen axial verschiebbar, nämlich zwischen einer Arretierposition A2 (z.B. Figur 6), in der kein Kontakt zum Spritzenkolben 14 besteht und einer Vorschubposition V2 (z.B. Figur 8), bei der der Spritzenkolben 14 in die Spritze 1, soweit dies vorgesehen ist, vollständig eingeschoben ist und die Injektionsflüssigkeit in der gewünschten Menge über die Injektionsnadel 13 in das Gewebe injiziert worden ist.

Die Bewegung des Stößels 4 zwischen Arretierposition A2 und Vorschubposition V2 wird im folgenden als "zweiter Hub", H2 (Hub II in Figur 1) als bezeichnet.

Es soll nochmals verdeutlicht werden, daß der erste Hub H1 des Schlittens 2 zum Axialvorschub der gesamten Spritze 1 und somit der Injektionsnadel 13 und damit zum Einstich der Injektionsnnadel 13 führt, wogegen erst der zweite Hub H2 die Axialbewegung des Stößels 4 dann zur Beaufschlagung des Spritzenkolbens 14 und zur Injektion der Injektionsflüssigkeit durch die (eingestochene) Injektionsnadel 13 führt.

Ein weiteres wesentliches Merkmal der erfindungsgemäßen Lösung besteht nun in der steuerungstechnischen Umsetzung dieser aneinander anschließenden ersten und zweiten Hübe H1,H2 zur Durchführung eines kompletten Einstich- und Injektionsablaufs:

Hierzu dient ein stiftartiges Kopplungselement 8 zwischen Schlitten 2 und Stößel 4, das während des ersten Hubs H1 des Schlittens 2 diesen und den Stößel 4 über ein erstes Rastelement 81 im Stößel 4 miteinander koppelt und nach Ablauf des ersten Hubes H1 mittels eines zweiten Rastelementes 73 im Gehäuse 7 die Kopplung löst, so daß dann der Stößel 4 alleine den zweiten Hub H2 durchführen kann.

Im dargestellten Ausführungsbeispiel besteht das Kopplungselement 8 aus einem bolzenähnlichen Stift mit konisch geformten Stirnseiten. Entsprechend dieser Formgebung der Stirnseiten bestehen die beiden Rastelemente aus im wesentlichen zu diesen Stirnseiten komplementären Vertiefungen 72 und 81 im Stößel 4 bzw. im Gehäuse 7, wobei insbesondere die entsprechenden konischen Abschrägungen in diesen Vertiefungen von Bedeutung sind.

In der Arretierposition A2 des Stößels 4 ist dieser mit dem Schlitten 2 dadurch über das Kopplungselement 8 verbunden, daß dessen nach innen zeigende Stirnseite in die Vertiefung 81 des Stößels 4 eingreift, wogegen auf der inneren Mantelfläche des gegenüberliegenden Abschnitts des Innenmantels des Gehäuses 7 keine Vertiefung ist. Damit sind Stößel 4 und Schlitten 2 in ihrer Bewegung zwangsgekoppelt und führen folglich den ersten Hub, der zum Einstich der Injektionsnadel 13 führt, gemeinsam aus.

Die Vorschubposition V1 des Schlittens 2 ist entsprechend durch die Positionierung der Vertiefung 72 im Gehäuse 7 definiert: Sobald der Schlitten 2 die gewählte Vorschubposition im Gehäuse 7 erreicht hat, erreicht das Kopplungselement 8 die Vertiefung 72 und gleitet infolge der konischen Formgebung mit seiner dieser zugewandten Stirnseite in diese hinein, mit der Folge, daß seine gegenüberliegende Stirnseite aus der entsprechenden Vertiefung 81 im Stößel 4 freikommt, wodurch die Zwangskopplung zwischen Stößel 4 und Schlitten 2 aufgehoben ist. Eine weitere Beaufschlagung des Stößels 4 in Richtung zur Spritze 1 hin führt dann also zur Durchführung des zweiten Hubes H2 zur Injektion der Injektionsflüssigkeit, bis die vordere Stirnseite des Führungsabschnittes 4A des Stößels 4 an der rückwärtigen Stirnseite des ersten Abschnittes 2A des Schlittens aufliegt, wodurch die Vorschubposition V2 des Stößels 4 definiert ist (Figuren 8 und 10).

Um diese beiden Hübe H1 und H2 in der beschriebenen Reihenfolge durch den Schlitten und den Stößel gemeinsam (Hub 1) und durch den Stößel alleine (Hub 2) durchführen zu können, wird eine Antriebseinrichtung benötigt, die den Stößel 4 auf seiner zur Gehäuserückwand 74 zeigenden Stirnseite beaufschlagt; beim dargestellten Ausführungsbeispiel ist hierzu eine koaxial zur Längsachse F angeordnete Spiralfeder 3 vorgesehen, deren eines Ende sich am Stößel 4 abstützt und deren anderes Ende an einem Halteblock 31 sitzt, der seinerseits in der Gehäuserückwand 74 gehalten ist.

Die Federkraft dieser Spiralfeder 3 ist so dimensioniert, daß sie zur Durchführung der beiden Hübe H1 und H2 ausreicht, d.h. insbesondere bis zur vollständigen Injektion der Injektionsflüssigkeit.

Die Spiralfeder 3 befindet sich folglich in ihrem gespannten Zustand, wenn Schlitten 2 und Stößel 4 sich in ihrer jeweiligen Arretierposition A1 bzw. A2 befinden.

Zwischen den Positionen von Stößel 4 und Schlitten 2 einerseits und den Positionen P1 bzw. P2 der Steuerhülse 6 andererseits ist eine Kopplung dahingehend definiert, daß weitere Funktionsbauteile vorgesehen sind, die gewährleisten, daß Schlitten 2 und Stößel 4 zwangsläufig in ihrer jeweiligen Arretierposition A1,A2 sich befinden müssen, wenn die Steuerhülse 6 sich in ihrer Öffnungsposition P2 befindet, oder umgekehrt ausgedrückt, daß erst, wenn die Steuerhülse 6 ihre Schließ- und Funktionsposition P1 eingenommen hat (wenn die Ladeöffnung L6 die Ladeöffnung L7 verschließt), Stößel 4 und Schlitten 2 ihre jeweilige Arretierpositionen verlassen und ihre oben definierten Vorschubpositionen V1 und V2 einnehmen können, bewegt von der Kraft der Spiralfeder 3.

Weiterhin ist vorgesehen, daß nach durchgeführter Injektion, wenn also die Steuerhülse 6 von ihrer Schließ- und Funktionsposition P1 wieder in ihre Öffnungs- und Sicherungsposition P2 verdreht wird, diese Drehbewegung über die eingangs angesprochenen Profilierungen auf der Mantelinnenseite MI eine entsprechende Rückführung von Stößel 4 und Schlitten 2 in ihre jeweiligen Arretierpositionen A1 bzw. A2 sorgt, anders ausgedrückt, daß der Zugriff in das Gehäuse 7 durch die Ladeöffnungen L6,L7 erst dann erfolgen kann, wenn sichergestellt ist, daß sich Stößel 4 und Schlitten 2 wieder in ihren jeweiligen Arretierpositionen befinden, in der dann naturgemäß die Feder 3 wieder gespannt und bereit zu einem neuen Injektionshub ist.

Die konstruktive Umsetzung dieses Steuerprinzips wird nun anhand von zwei Ausführungsbeispielen im einzelnen erläutert:

Eine bevorzugte Umsetzung der steuerungsmäßigen Kopplung zwischen der Steuerhülse 6 und dem Schlitten 4 ist in den Figuren 11 und 12 dargestellt; bei dieser Umsetzung sind zwei baugleiche Hubbolzen 42 und 43 vorgesehen, die mit einer Profilierung in Form einer sägezahnähnlichen Steuerkurve mit den Steuerkurvenabschnitten 68,69 auf der Mantelinnenseite MI der Steuerhülse 6 zusammenwirken. Dies ist eine konstruktiv einfache Lösung, bei der Reibungseffekte zwischen den Hubbolzen 42,43 und der Mantelinnenseite MI nur minimal sind; durch die senkrechten Abfallflanken der Abschnitte 68,69 der Steuerkurve ist eine eindeutige Funktionszuordnung zur Drehrichtung DR gegeben. Die Verminderung von Reibungseinflüssen führt zu einem geringen Betätigungsdrehmoment und damit zu einem komfortablen Handhabungsempfinden.

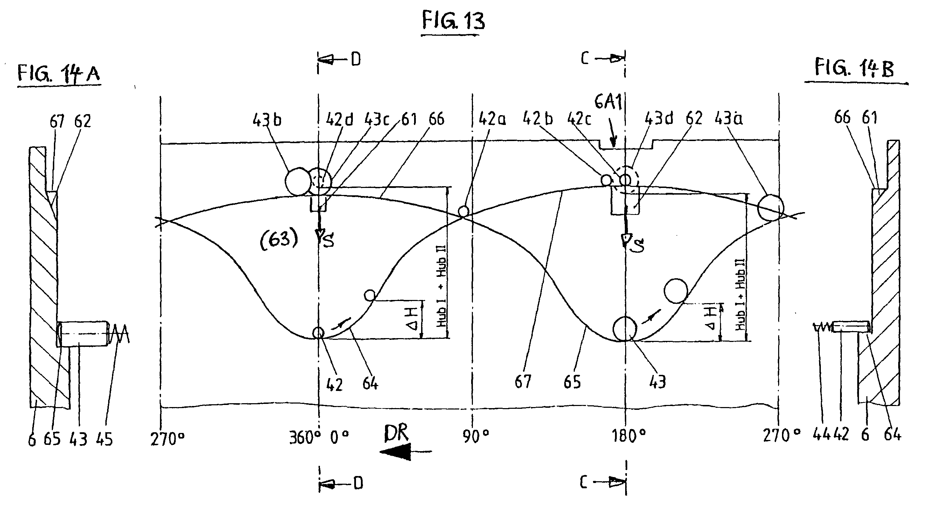

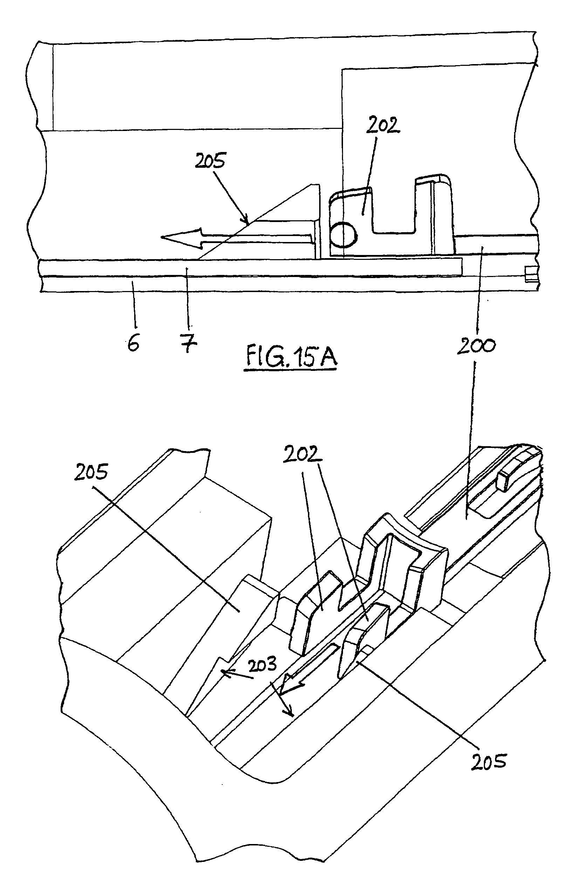

Eine alternative Umsetzung der steuerungsmäßigen Kopplung zwischen der Steuerhülse 6 und dem Schlitten 4 ist in den Figuren 13 und 14 dargestellt, wobei für gleiche Bauteile auch die gleichen Bezugszeichen verwendet werden.

Bei beiden Umsetzungen dieser Kopplung wird diese über quer zur Längsachse F im rückwärtigen Teil 4A des Stößels 4 gehaltene Hubbolzen 42 und 43 gleichen oder unterschiedlichen Durchmessers erreicht, die so dimensioniert und im Stößel 4 orientiert sind, daß ein Kraftschluß zwischen den Hubbolzen 42,43 einerseits und den Steuerkurvenabschnitten 64 bis 67 (Fig. 13 und 14) bzw. 68,69 (Fig. 11 und 12) erreicht wird. Dadurch ist es möglich, durch Verdrehen der Steuerhülse 6 das hierbei aufgebrachte Drehmoment über die jeweiligen Steuerkurven in eine lineare Rückstellkraft des Stößels 4 gegen die Spannkraft der Spiralfeder 3 umzusetzen, so daß der Stößel 4 und der Schlitten 2 im Gehäuse 7 so weit zurückgeschoben werden, bis sie wieder ihre jeweiligen Arretierpositionen A1,A2 erreicht haben. Der dabei ausgeführte gesamte Gegenhub ΔH entspricht dabei in seinem Wert der Summe der beiden Hübe H1 und H2 von Stößel 4 und Schlitten 2. Die in der Abwicklung der Mantelinnenfläche MI in Figur 13 dargestellte Form der beiden Steuerkurven ist darin begründet, daß die Umsetzung des Drehmomentes beim Verdrehen der Steuerhülse 6 über den gesamten Gegenhub ΔH mit möglichst konstantem Drehmoment erfolgen sollte, um die Handhabung der Injektionsvorrichtung zu erleichtern. Durch die gegenüberliegende Anordnung der beiden Hubbolzen 42,43 wird hierbei der von der Steuerhülse 6 erzeugte Gegenhub ΔH so auf Stößel bzw. Schlitten übertragen, daß Kippmomente auf Stößel und/oder Schlitten, die zu einem Verkanten oder Verklemmen führen könnten, zuverlässig ausgeschlossen werden. Die Verteilung der Drehmomentübertragung auf die beiden Hubbolzen 42,43 ermöglicht eine kurze Führung des Stößels 4 und somit eine gedrungene, kompakte Bauweise der Injektionsvorrichtung in diesem Bereich.

Vorzugsweise ist die Gestaltung der Steuerkurvenabschnitte 64 bis 67 bzw. 68,69 so gewählt, daß eine Entformung der Steuerhülse 6 in einem Spritzgießwerkzeug problemlos erfolgen kann.

Da beim Ausführungsbeispiel gemäß Figuren 13 und 14 der Durchmesser des ersten Hubbolzens 42 signifikant kleiner ist als der Durchmesser des zweiten Hubbolzens 43, schneiden sich die zugeordneten Steuerkurvenabschnitte 64 und 65 nicht bei 90°, sondern etwas vorher. Dieser Versatz des Schnittpunktes bewirkt, daß derbei diesem Ausführungsbeispiel federbelastete Hubbolzen 42 infolge der durch die Feder 44 auf ihn wirkenden Kraft eine axiale Bewegung durchführt und in der Stellung 42a bereits den Steuerkurvenabschnitt 64 verläßt und auf den anschließende Steuerkurvenabschnitt 67 gleitet. Bei dieser Stellung der Steuerhülse 6 befindet sich der ebenfalls federbelastete (Feder 45) Hubbolzen 43 noch auf dem Steuerkurvenabschnitt 65. Dadurch ist gewährleistet, daß sich immer nur ein Hubbolzen im Übergang von einem Steuerkurvenabschnitt zum anschließenden anderen befindet, so daß immer eine gesicherte Kraftübertragung von der Steuerhülse 6 zum Stößel 4 über zumindest einen der beiden Hubbolzen 42,43 erfolgt.

Unterhalb der in Figur 13 dargestellten Position 42d bzw. 43d sind die Steuerkurvenabschnitte 66,67 mit Gleitrampen 61,62 versehen, über die die Hubbolzen 42,43 in ihre Ausgangslage zurückkehren (parallele Pfeile S in Fig. 13).

Eine weitere konstruktive Umsetzung des Steuerprinzips mittels der Steuerhülse 6 betrifft die Sicherung der Injektionsvorrichtung in der Arretierposition des Stößels 4, abhängig von der Winkelstellung der Steuerhülse 6:

Als Sicherungsmittel sind hierzu an der Rückseite des Führungsabschnittes 4A des Stößels 4 zwei gegenüberliegende Sicherungsklinken 41 angeformt, die in der Öffnungs- und Sicherungsposition P2 der Steuerhülse 6 mit zwei quer zur Funktionsrichtung F verschiebbaren Steuerklinken 53 und 54 einer Auslöseeinrichtung in lösbarem Eingriff stehen und so den Stößel 4 gegen die Vorspannung der Spiralfeder 3 in seiner Arretierposition A2 halten. Beim dargestellten Ausführungsbeispiel besteht der beschriebene Eingriff darin, daß die beiden Sicherungsklinken 41 in gleiche Richtung zeigende Nasen aufweisen, die über die rückwärtigen Flächen der Steuerklinken 53,54 in die Arretierposition gleiten, wozu die Steuerklinken rampenartige Anschrägungen aufweisen, so daß der Eingriff ein elastisches Einschnappen der Sicherungsklinken 41 über die Steuerklinken 53,54 bewirkt, wie dies in Figur 1 dargestellt ist.

Die Steuerklinken 53 und 54 sind Teil einer Auslöseeinrichtung, die durch das Gehäuse 7 in dessen Handhabungsabschnitt 7A nach außen geführt ist und als Betätigungstaste 5 ausgebildet ist. Diese Betätigungstaste 5 mit den Steuerklinken 53 und 54 ist gegen eine Feder 51 im Gehäuse 7 bewegbar, die dafür sorgt, daß der in Figur 1 dargestellte Sicherungseingriff zwischen Stößel 4 und Auslöseeinrichtung erhalten bleibt.

Das Zusammenwirken zwischen Betätigungstaste 5 und Steuerhülse 6 ist nun so, daß die Betätigungstaste 5 nur quer zur Längsachse F verschoben werden kann (und damit die Sicherungsklinken 41 freigegeben werden), wenn die Steuerhülse 6 sich in ihrer Schließ- und Funktionsposition P1 befindet. Da diese Schließ- und Funktionsposition P1 eindeutig durch die Winkelstellung der Steuerhülse 6 auf dem Gehäuse 7 definiert ist (vergl. Figur 2), ist diese steuerungstechnische Kopplung zwischen Betätigungstaste 5 und Steuerhülse 6 einfach dadurch erreicht, daß sich der hintere Randbereich des Steuerabschnitts 6A der Steuerhülse 6 rückseitig über eine Nase 5A der Betätigungstaste 5 erstreckt (diese hintergreift), und nur in dem entsprechenden Winkelbereich, der der Schließ- und Funktionsposition P1 entspricht, eine randseitige Ausnehmung 6A1 aufweist (Figur 11), durch die in der Schließ- und Funktionsposition P1 die Nase 5A der Betätigungstaste 5 passieren kann. In der Schließ- und Funktionsposition P1 der Steuerhülse 6 kann folglich die Arretierposition A2 des Stößels 4 aufgehoben werden, indem die Betätigungstaste 5 in das Gehäuse 7 gedrückt wird und damit die Steuerklinken 53,54 die beiden Sicherungsklinken 41 freigeben, wodurch dann sofort die Spiralfeder 3 ihre Kraft entfalten kann und die beiden Hübe H1 und H2 nacheinander ausgeführt werden, wie dies eingangs beschrieben ist.

Zur eindeutigen Definition der beiden Positionen P1 und P2 der Steuerhülse 6 ist ferner vorgesehen, daß der vordere, sich konisch verjüngende Führungsabschnitt 6C der Steuerhülse 6 gegenüberliegende, nach innen gerichtete, schalenförmige Ausnehmungen 77A und 77B aufweist, die in Wechselwirkung stehen mit einem federbeaufschlagten Rastelement 77, das im Gehäuse 7 gehalten ist. Bei der in Figur 1 dargestellten Öffnungs- und Sicherungsposition P2 der Steuerhülse 6 befindet sich das Rastelement 77 im Eingriff mit der Ausnehmung 77A, bei der beispielsweise in Figur 8 dargestellten Schließ- und Funktionsposition P1 befindet sich demnach das Rastelement 77 im Eingriff mit der Vertiefung 77B.

Die von diesem Rastmechanismus hervorgerufene kurzzeitige, aktive Verdrehung der Steuerhülse 6 bewirkt somit eine sensorische Wahrnehmung der beiden Grundpositionen P1 und P2 der Steuerhülse 6 für den Benutzer und dient damit gewissermaßen auch als Rückmeldung des aktuellen Funktionszustandes der Injektionsvorrichtung, was die Bedienungssicherheit weiter erhöht.

Mit den oben beschriebenen Funktionsbauteilen ist bereits eine ordnungsgemäße und zuverlässige Funktion der Injektionsvorrichtung möglich, zur weiteren Ausgestaltung und Verbesserung sind jedoch noch weitere Einrichtungen vorgesehen, die im folgenden ergänzend noch kurz vorgestellt werden:

Im Schlitten 2 ist ein Rotationsdämpfer 100 handelsüblicher Bauart vorgesehen, der über eine Zahnstange 101 mit dem Gehäuse 7 verbunden ist. Es handelt sich hierbei um einen Rotationsdämpfer, wie er beispielsweise unter der Produktbezeichnung FRT-C2 von der Firma ACE Stoßdämpfer GmbH in D-40764 Langenfeld erhältlich ist. Die Wirkungsweise eines solchen Rotationsdämpfers 100 besteht im wesentlichen darin, daß die Relativbewegung zwischen dem Körper und der Zahnstange 101 durch Zwischenschaltung eines Mittels bestimmter Viskosität erfolgt, so daß eine zwischen diesen beiden Komponenten wirkende Kraft eine geringere Beschleunigung bei der resultierenden Linearverschiebung hervorruft. Die Funktion des Rotationsdämpfers 100 ist bei der Injektionsvorrichtung so gewählt, daß die Beschleunigung des Schlittens 2 während des ersten Hubes H1 (also bei hoher Spannkraft der Feder 3) auf einen gewünschten Wert begrenzt wird, der einerseits noch einen sicheren, schnellen Einstich der Injektionsnadel 13 in das Gewebe gewährleistet, andererseits aber allzu deutliche Anschlageffekte, wie Erschütterungen und Vibrationen am Ende des ersten Hubes H1 vermeidet, so daß eine insgesamt "geschmeidigere" Betriebsweise bei der Injektion erzielt wird.

Zur Erleichterung der Handhabung bei der Entnahme der Spritze 1 nach abgeschlossenem Injektionsvorgang ist eine Auswurfeinrichtung gegenüber den Ladeöffnungen L6,L7 des Gehäuses 7 bzw. der Steuerhülse 6 (also insofern "unterhalb" der Spritze 1) vorgesehen, die bei der Öffnung der Injektionsvorrichtung (Verdrehung der Steuerhülse 6 von der Schließ- und Funktionsposition P1 in ihre Öffnungs- und Sicherungsposition P2) in dem Sinne aktiviert wird, daß sie das rückseitige Ende der Spritze 1 dem Benutzer sozusagen entgegen hebt und die Entnahme erleichtert. Auch hierbei soll wieder die Funktion der Steuerhülse 6 als Steuermittel in dem Sinne hervorgehoben werden, daß bestimmte Funktionen oder Zusatzfunktionen ausschließlich durch die Betätigung der Steuerhülse aktiviert oder ermöglicht werden.

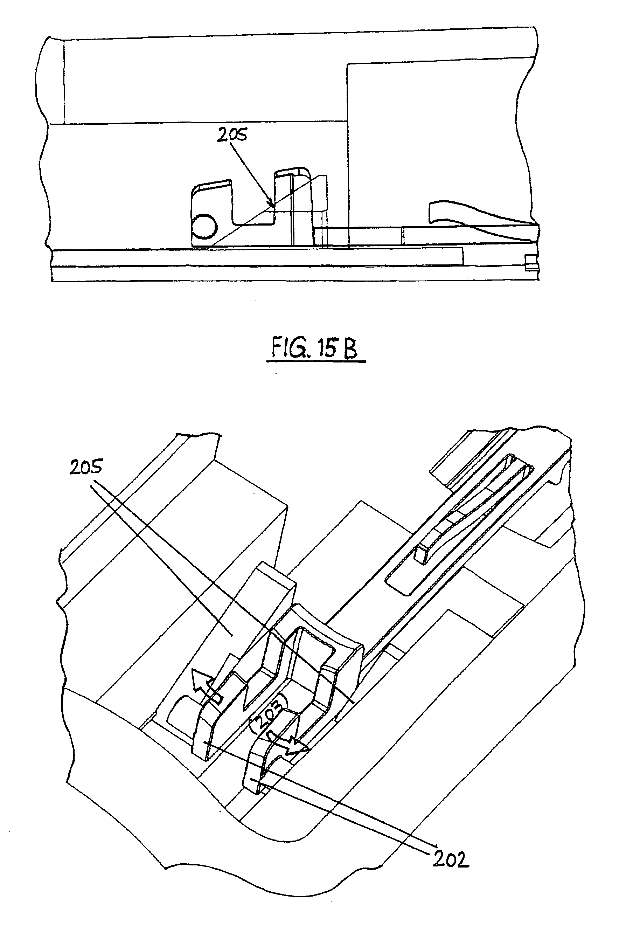

Die Auswurfeinrichtung (insbesondere Figur 15 hierzu) beinhaltet zunächst einen Auswurfhebel 200, dessen eines Schenkelende mittels eines Stiftes 201 am Schlitten 2 derart schwenkbar gehalten ist, daß sein anderes, um 90° abgewinkeltes Schenkelende in Richtung zur Spritze 1 hin bewegbar ist. Hierzu weist der Schlitten 2 eine entsprechende Ausnehmung oder Abschrägung 2C auf, die gleichzeitig auch den Schwenkwinkel des Auswurfhebels 200 definiert. Der Auswurfhebel 200 weist in der gleichen Ebene wie sein erster Schenkel einen Steuernocken auf, der aus zwei gabelähnlich angeordneten Gleitnocken 202 besteht, die in einem sich in Hubrichtung verengenden Führungskanal 203 geführt sind, bis ihre hakenähnlich nach außen zeigenden Enden in Eingriff mit dem unteren Ende einer Führungsbahn 205 kommen, wenn der erste Hub beendet ist. Die Führungsbahn 205 steigt in Richtung zur Längsachse F hin rampenähnlich entgegen der Injektionsrichtung an und ist derart positioniert und orientiert, daß bei der Drehung der Steuerhülse 6 in ihre Öffnungs- und Sicherungsposition P2 und somit der Bewegung des Schlittens 2 zurück in seine Arretierposition A1, die beiden Gleitnocken auf diese Führungsbahn 205 gelangen und damit der Auswurfhebel 200 nach oben geführt wird, so daß die Auswurfbewegung abgeschlossen ist, wenn die Steuerhülse 6 ihre Öffnungs- und Sicherungsposition P2 erreicht hat.

Als weitere zusätzliche Einrichtung weist die Injektionsvorrichtung eine Signaleinrichtung auf, die nach erfolgter Injektion am Ende des zweiten Hubes H2 betätigt wird. Hierbei ist vorgesehen, daß diese Signaleinrichtung ausschließlich aus mechanisch wirkenden Bauelementen besteht und mittels einer Glocke 306 ein akustisches Signal abgegeben wird, zu dessen Erzeugung ein quer zur Längsachse F bewegbarer Schlagstift 305 dient, der den Randbereich der Glocke 306 impulsartig beaufschlagt.

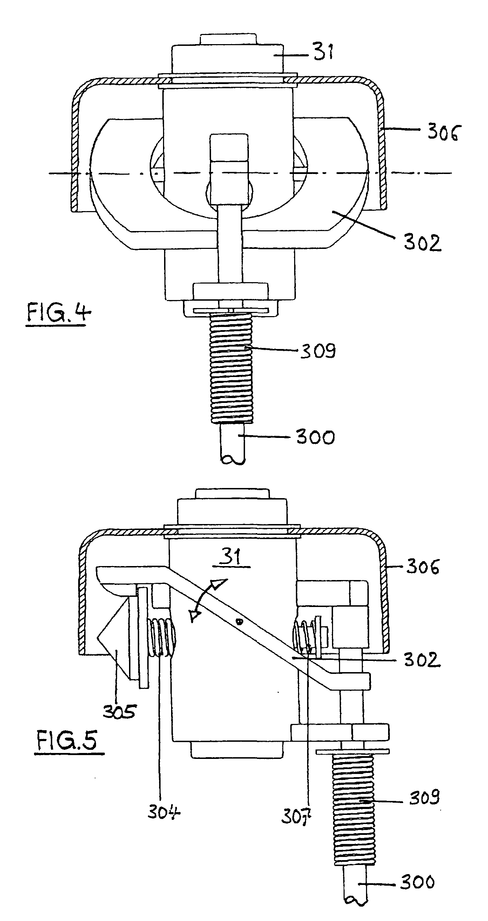

Der Detailaufbau der Signaleinrichtung ist in den Figuren 3,4 und 5 dargestellt:

In einer Bohrung des Stößels 4 ist das eine Ende einer Zugstange 300 beweglich gehalten, deren anderes Ende an einem kragenähnlichen Bügel 302 angreift, der am Halteblock 31 gehalten ist, der in der Rückseite 74 des Gehäuses befestigt ist und der an seiner Vorderseite als Gegenlager der Spiralfeder 3 dient. Mittels einer Umfangsnut ist an diesem Grundkörper 31 auch die Glocke 306 gehalten, so daß sie den Bügel 302 überdeckt. Die Drehachse 303 des Bügels 302 verläuft dabei durch die Längsachse F senkrecht zur Zeichenebene der Figur 3. An seinem von der Zugstange 300 abgewandten Ende übergreift der Bügel 302 eine Randnut des Schlagstiftes 305, der in der Arretierposition von einer Zunge 308 des Stößels 4 fixiert ist. Der Schlagstift 305 ist mittels zweier Federn 304 und 307 in der Zeichenebene der Figur 3 kraftbeaufschlagt gehalten. Die Zugstange 300 ist mit einer Druckfeder 309 umgeben, die dafür sorgt, daß der Bügel 302 in der in Figur 3 dargestellten Rastposition verbleibt.

Die Länge der Zugstange 300 ist so gewählt, daß am Ende der Injektion (Figur 10) der Stößel 4 über einen Anschlag 301 die Zugstange 300 beaufschlagt, so daß diese nun ihrerseits den Bügel 302 verschwenkt, worauf das gegenüberliegende Ende des Bügels 302 die Umfangsrastung des Schlagstiftes 305 freigibt, so daß der (von der Zunge 308 bereits freigegebene) Schlagstift 305 unter der koordinierten Kraft der beiden Federn 304,307 gegen den inneren Randbereich der Glocke 306 geschleudert wird, wie dies in Figur 10 dargestellt ist. Die Federkonstanten der Federn 304 und 307 sind hierbei so dimensioniert, daß zunächst sichergestellt ist, daß der Schlagstift 305 die Glocke 306 erreicht, um das akustische Signal auszulösen, dann jedoch die Feder 307 den Schlagstift 305 wieder zurückzieht, so daß die Glocke ohne Dämpfungseffekte seitens des aufliegenden Schlagstiftes 305 frei ausschwingen kann und den typischen Ton einer klöppelbetätigten Glocke erzeugen kann. Bei Rückführung des Stößels 4 in seine Arretierposition gleitet das abgeschrägte Ende der Zunge 308 wieder auf die Spitze des Schlagstiftes 305 und drückt diesen so weit zurück, bis die Sperrklinke des dann wieder von der Druckfeder 309 beaufschlagten Bügels 302 die Randnut des Schlagstiftes 305 übergreift und diesen sichert.

Nachdem die baulichen Voraussetzungen nun ausführlich dargelegt wurden, soll deren Funktion anhand eines vollständigen Injektionsvorgangs stufenweise noch kurz beschrieben werden:

Als Ausgangsposition ist die Stellung und Anordnung der Bauteile gewählt, wie sie in den Figuren 1 bis 3 dargestellt ist:

Hierbei befindet sich die Steuerhülse 6 in ihrer Öffnungs- und Sicherungsposition P2, d.h., durch die Ladeöffnungen L6 und L7 kann eine Spritze 1 in ihre dafür im Schlitten 2 vorgesehene Position eingelegt werden. In der Öffnungs- und Sicherungsposition P2 ist dabei die Steuerhülse 6 durch das in die Ausnehmung 77A eintauchende Rastelement 77 gehalten.

Die beiden Hubbolzen 42,43 befinden sich in ihrer Position B (Figur 12) bzw. in der in Figur 13 dargestellten obersten Position 42c,43c auf ihren zugeordneten Steuerkurvenabschnitten.

Stößel 4 und Schlitten 2 befinden sich in ihrer rückwärtigen Arretierposition A1 bzw. A2, da die Sicherungsklinken 41 in Eingriff mit den beiden Steuerklinken 53 und 54 stehen und die Aktivierung der Auslöseeinrichtung nicht möglich ist, da die Nase 5A der Betätigungstaste vom Randbereich des zylindrischen Steuerabschnitts 6A der Steuerhülse 6 gesperrt ist.

Die Zunge 308 am Führungsabschnitt 4A des Stößels 4 ist mit ihrer rückwärtigen Stirnseite in Kontakt mit dem kegelförmigen Schlagstift 305, die Sperrklinke des Bügels 302 wird durch die Kraft der Feder 309 über den umlaufenden Rand des Schlagstiftes 305 gepreßt und hält diesen fest. Die Aktivierung der Glocke 306 ist somit in dieser Position ebenfalls nicht möglich. Die Feder 3 befindet sich in ihrem zusammengedrückten, also aktionsbereiten Zustand.

Wenn die Spritze 1 mit ihrer Schutzkappe 11 in den Schlitten 2 eingelegt und durch ihren Spritzenbund 12 fixiert ist, wird die Steuerhülse 6 mit der einen Hand des Benutzers um 180° gedreht, wobei die andere Hand des Benutzers den Handhabungsabschnitt 7A des Gehäuses 7 festhält. Dadurch wird die Ladeöffnung L7 des Gehäuses 7 von der Steuerhülse 6 verschlossen und es ist folglich die in Figur 9 dargestellte Schließ- und Funktionsposition P1 der Steuerhülse 6 erreicht. In dieser Position taucht dann das Kontaktelement 77 in die Vertiefung 77B in der Steuerhülse 6 und die Hubbolzen 42,43 befinden sich auf Pos. C (Fig. 12) bzw. Pos. 42d;43d (Fig. 13). Gleichzeitig kommt die Ausnehmung 6A1 im hinteren Randbereich des zylindrischen Steuerabschnittes 6A in den Bereich der Nase 5A der Betätigungstaste, so daß folglich in der Schließ- und Funktionsposition P1 die Injektionsvorrichtung benutzt werden kann.

Hierzu wird zunächst die Schutzkappe 11 von der Nadel 13 der Spritze 1 abgenommen und die Injektionsvorrichtung wird auf die vorgesehene Injektionsstelle aufgesetzt. Nach ordnungsgemäßer Positionierung wird dann die Auslöseeinrichtung aktiviert, d.h. die Betätigungstaste 5 wird in das Gehäuse 7 gedrückt, wodurch nach einem kurzen Weg im Millimeter-Bereich die Steuerklinken 53,54 die beiden Sicherungsklinken 41 freigeben. Sobald dies erfolgt ist, kann die Feder 3 ihre Wirkung entfalten und drückt die über das Kopplungselement 8 noch kraftschlüssig miteinander verbundenen Stößel 4 und Schlitten 2 nach vorne, d.h. es wird unter der verzögernden Wirkung des Rotationsdämpfers 100 der erste Hub H1 ausgeführt, der zum Einstechen der Injektionsnadel 13 in das Gewebe führt. Während dieser Bewegung des Schlittens 2 bewegt sich der Auswurfhebel 200, der im Schlitten 2 über den Stift 201 gehalten ist, mit seinen Gleitnocken 202 auf der Bahn 203. Durch die sich verengende Bahn 203 werden die beiden Schenkel 204 nach innen gedrückt, so daß gegen Ende des ersten Hubes eine Spannung erzeugt wird, durch die die Gleitnocken 202 nach außen springen und mit der Führungsbahn 205 in Eingriff kommen.

Bei dem in den Figuren 13 und 14 dargestellten Ausführungsbeispiel gleiten am Beginn des ersten Hubes H1 außerdem die durch die Federn 44,45 belasteten Hubbolzen 42,43 über die Gleitrampen 61,62 in den Freiraum 63. Das Ende des ersten Hubes H1, den Schlitten 2 und Stößel 4 gemeinsam ausführen, ist, wie oben erläutert, dadurch definiert, daß das Kopplungselement 8 bei seinem Entlanggleiten auf der Innenfläche des Gehäuses 7 schließlich das als Vertiefung 72 ausgebildete Rastelement erreicht. Aufgrund der Kraftumsetzung über die angeschrägten Flächen der Vertiefungen gleitet das Kopplungselement 8 in die Vertiefung 72, wodurch der Formschluß und Kraftschluß zwischen Stößel 4 und Schlitten 2 aufgehoben ist und nunmehr der Schlitten 2 in seiner vorderen Endposition im Gehäuse 7 angekommen und formschlüssig fixiert wird. Damit hat auch die Injektionsnadel 13 ihre vorderste Position in der Längsachse F erreicht.

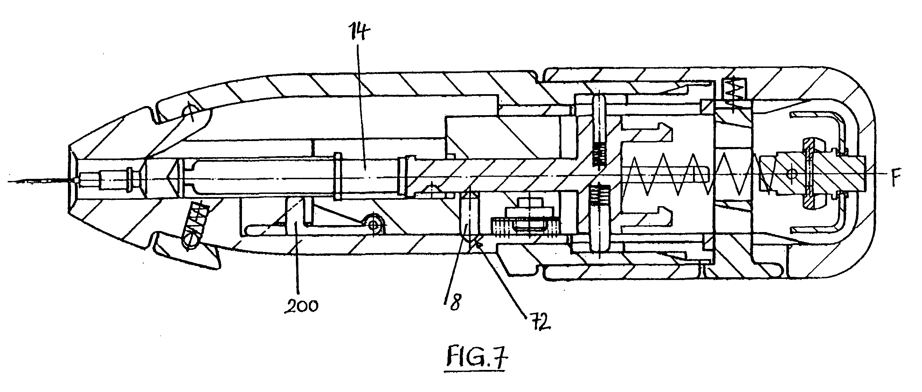

Unter der Wirkung der Feder 3 führt nun der Stößel 4 alleine den zweiten Hub H2 aus, in Figur 6 ist unmittelbar der Beginn dieses zweiten Hubes dargestellt, wenn sich die Ausnehmung 81 im Stößel 4 vom nunmehr im Gehäuse 7 gehaltenen Kopplungselement 8 entfernt. Die Frontseite des Stößels 4 beaufschlagt nun im weiteren Ablauf des zweiten Hubes den Kolben 14 der Spritze 1, drückt diesen in die Spritze und bewirkt somit die Injektion der Injektionsflüssigkeit; diese Position gegen Ende des zweiten Hubes ist in Figur 7 dargestellt.

Am Ende der Injektion, wenn also der Stößel 4 seine vordere Endposition erreicht hat, betätigt er über den Anschlag 301 (Figur 10) die Zugstange 300, wodurch über den Hebel 302 die Verrastung des Schlagstiftes 305 freigegeben wird. Unter der Wirkung der Feder 304 schlägt dieser auf den inneren Randbereich der Glocke 306 auf, so daß der eingangs erwähnte, vom Benutzer des Gerätes deutlich wahrnehmbare Ton erzeugt wird. Unter der Gegenwirkung der Feder 307 wird der Schlagstift 305 unmittelbar nach dieser Aktion von der Glocke zurückgezogen, so daß die Glocke ausschwingen kann.

Der Benutzer hat nun die Information über die Beendigung des Injektionsvorganges erhalten und kann folglich die Injektionsvorrichtung von der Injektionsstelle abheben und die Injektionsnadel 13 aus dem Gewebe herausziehen.

Zur Entnahme der Spritze 1 wird nun die Steuerhülse 6 wiederum um 180° von ihrer Schließ- und Funktionsposition P1 in die Öffnungs- und Sicherungsposition P2 (Figur 2) bewegt, so daß die Spritze 1 wieder zugänglich wird. Diese Verdrehung der Steuerhülse 6 hat zunächst die Wirkung, daß durch die Steuerkurvenabschnitte 64 bis 67 bzw. 68,69 im Zusammenwirken mit den Hubbolzen 42,43 die Drehbewegung um 180° in den Gegenhub ΔH des Stößels 4 umgesetzt wird (der durch nicht dargestellte Vorkehrungen an einer radialen Bewegung gehindert wird). Erreicht der rückwärtige Anschlag 46 hierbei wieder den Schlitten 2, gleitet das Kopplungselement 8 wieder außer Eingriff mit dem Gehäuse 7 und der Schlitten 2 wird wieder an die Bewegung des Stößels 4 gekoppelt.

Während dieses ersten Abschnittes der Rückbewegung des Schlittens 2 bewegen sich die Gleitnocken des Auswurfhebels 200 mit dem Schlitten 2 auf der Führungsbahn 205 schräg nach oben, so daß in diesem Bereich die Verdrehung der Steuerhülse 6 in eine Drehbewegung des Auswurfhebels 200 umgesetzt wird, der die Spritze 1 schließlich untergreift und hochhebt, wenn die Drehung der Steuerhülse 6 beendet ist.

Gegen Ende dieser Drehbewegung gleiten die abgeschrägten Enden der Sicherungsklinken 41 auf die entsprechenden Abschrägungen der beiden Steuerklinken 53 und 54, bis am Ende dieses Vorgangs die federbeaufschlagte Auslöseeinrichtung mit ihren Steuerklinken 53,54 wieder hinter den rückseitigen Flanken der beiden Sicherungsklinken 41 einrastet, bevor dann schließlich wieder die beiden Arretierpositionen A1 bzw. A2 von Stößel 4 und Schlitten 2 erreicht sind.

Gleichzeitig mit diesem Vorgang taucht auch wieder die Zunge 308 durch die zugehörige Öffnung in der Auslöseeinrichtung. Durch die korrespondierend abgeschrägten Kontaktflächen zwischen Zunge 308 und Schlagstift 305 wird die Axialbewegung der Zunge 308 in eine entsprechende Querbewegung des Schlagstiftes 305 umgesetzt, bis dieser schließlich wieder eine Position erreicht, wo die inzwischen durch die Druckfeder 309 wieder druckbelastete Zugstange den Hebel 302 so verschwenken kann, daß dessen gegenüberliegendes Ende wieder auf dem Umfangsrand des Schlagstiftes 305 verrastet.

Die Spritze 1 kann nun entnommen werden und die Injektionsvorrichtung in diesem Zustand bis zum nächsten Gebrauch aufbewahrt werden. Ein Verdrehen der Steuerhülse 6 ist jedoch ebenfalls möglich, so daß die oben beschriebenen Vorgänge auch "leer" durchgeführt werden können. In jedem Fall bewirkt die Verdrehung der Steuerhülse 6 um 180° in ihre Öffnungs- und Sicherungsposition P2 die Beaufschlagung des Stößels 4 und des Schlittens 2 und deren Rückführung in ihre Arretierungsposition, so daß die Injektionsvorrichtung dann zur Aufnahme einer neuen Spritze 1 bereit ist.

Zur Verdeutlichung der letztgenannten Umsetzung der Verdrehbewegung der Steuerhülse 6 in die Axialverschiebung von Stößel und Schlitten zurück in ihre Arretierposition sollen diese Abläufe anhand der Figuren 11 bis 14 nochmals erläutert werden:

Beim bevorzugten Ausführungsbeispiel (Fig. 11 und 12) befinden sich nach erfolgter Injektion die beiden Hubbolzen 42,43 in ihrer tiefsten Position D; bei Drehung der Steuerhülse 6 in Richtung DR gleiten die beiden Hubbolzen in Richtung der schräg nach oben gerichteten Pfeile auf "ihrem" Abschnitt 68,69 der Steuerkurve und führen hierbei den Gegenhub ΔH aus. Nach Erreichen des Scheitelpunktes der Steuerkurvenabschnitte 68,69 (Position B) nach einer Drehung um ca. 180° sind wiederum die Arretierpositionen von Stößel 4 und Schlitten 2 erreicht. Bei Weiterdrehung der Steuerhülse 6 um wiederum 180° gelangen die Hubbolzen dann in die Position C, in der auch die Verrastung mittels Rastelement 77/Ausnehmung 77A bewirkt ist und der Spritzenwechsel vorgenommen werden kann. Nach erneuter Aktivierung der Injektionsvorrichtung bewegen sich die beiden Hubbolzen (Pfeile S) über die senkrechte Flanke ihres Steuerkurvenabschnitts 68,69 nach Beendigung des Injektionsvorgangs wieder in ihre Position D.

Beim zweiten Ausführungsbeispiel mit zwei Steuerkurven (Fig. 13 und 14) befindet sich nach erfolgter Injektion, also am Ende der beiden Hübe H1 und H2, der erste Hubbolzen 42 am tiefsten Punkt seines zugeordneten Steuerkurvenabschnitts 64 und entsprechend der zweite Hubbolzen 43 am tiefsten Punkt seines zugeordneten Steuerkurvenabschnitts 65. Wird die Steuerhülse 6 nun in Richtung des Pfeiles DR gedreht, gleiten die beiden Hubbolzen 42,43 relativ gesehen in die entgegengesetzte Richtung (kleine, schräg nach oben gerichtete Pfeile) auf den Steuerkurvenabschnitten 64 und 65 und führen dabei den Gegenhub ΔH aus, der den beiden Hüben H1 und H2 bei der Durchführung der Injektion entgegengerichtet ist. Pro Drehwinkeleinheit der Steuerhülse 6 verläuft der Steuerkurvenabschnitt 64 bzw. 65 relativ steil, da auch hier die Feder 3 sich noch weitgehend in ihrer entspannten Position befindet und somit bei der Verdrehung der Steuerhülse 6 dem entsprechenden Gegenhub von Schlitten 2 und Stößel 4 nur wenig Kraft entgegensetzt. Die Formgebung der Steuerkurvenabschnitte 64 und 65 ist somit ein unmittelbares Spiegelbild der zunehmenden, dem Gegenhub ΔH entgegenwirkenden Kraft der Feder 3, wobei eine Optimierung insofern erreicht werden kann, als pro Winkeleinheit der Verdrehung der Steuerhülse 6 ein annähernd gleiches Drehmoment vom Benutzer ausgeübt werden muß, was den Bedienungskomfort gewährleistet. Dies bedeutet umgekehrt, daß eine Verdrehung der Steuerhülse 6 um einen festen Drehwinkel am Anfang der Verdrehung zu einem größeren entsprechenden Gegenhub führt als am Ende der Verdrehungsbewegung, wo die Feder 3 mit ihrer nahezu vollen Spannkraft einen sehr flachen Verlauf der Steuerkurvenabschnitte 66,67 verlangt.

Durch den Versatz der Schnittpunkte der Steuerkurvenabschnitte 64,67 bzw. 65,66 führt der erste Hubbolzen 42 infolge der durch die Feder 44 auf ihn wirkenden Kraft eine axiale Bewegung durch, bei der er in der Stellung 42a seinen Steuerkurvenabschnitt 64 verläßt und auf den Steuerkurvenabschnitt 67 gleitet. Zu diesem Übergangszeitpunkt befindet sich der zweite Hubbolzen 43 jedoch noch auf seinem Steuerkurvenabschnitt 65, so daß die Drehmomentübertragung von der Steuerhülse 6 zum Stößel 4 gewährleistet bleibt.

In der Stellung 42b,43b, nach einer Drehung der Steuerhülse 6 von etwas weniger als 180°, haben die beiden Hubbolzen 42,43 den erforderlichen Gegenhub ΔH des Stößels 4 erzeugt, der in seinem Betrag der Summe der beiden Hübe H1 und H2 entspricht, wie dies in Figur 13 dargestellt ist und die Arretierposition von Stößel 4 und damit Schlitten 2 sind erreicht. Die Steuerhülse wird dann noch etwas weitergedreht, bis dann erst die oben schon erwähnte Verrastung des Rastelementes 77 wieder in der Ausnehmung 77A bewirkt wird, wodurch auch dem Benutzer mitgeteilt wird, daß nunmehr die Arretierung wieder sichergestellt ist und die leere Spritze 1 entnommen und gegebenenfalls eine neue wieder eingelegt werden kann. Die beiden Hubbolzen 42,43 nehmen die Position 42c,43c ein.

Beim Übergang der Steuerhülse 6 von ihrer Öffnungs- und Sicherungsposition P2 in ihre Schließ- und Funktionsposition P1 werden die beiden Hubbolzen 42 und 43 wieder um 180° in ihre ursprüngliche Winkelposition bei 42d bzw. 43d geführt, jedoch um die Summe der beiden Hübe H1 und H2 versetzt; erst bei der nunmehr wieder möglichen Aktivierung der Injektionsvorrichtung durch die Auslöseeinrichtung erreichen die beiden Hubbolzen über die Gleitrampen 61,62 wieder die am Eingang der Funktionsbeschreibung vorausgesetzte Position am unteren Scheitelpunkt ihrer zugeordneten Steuerkurvenabschnitte nach Beendigung der Injektion.

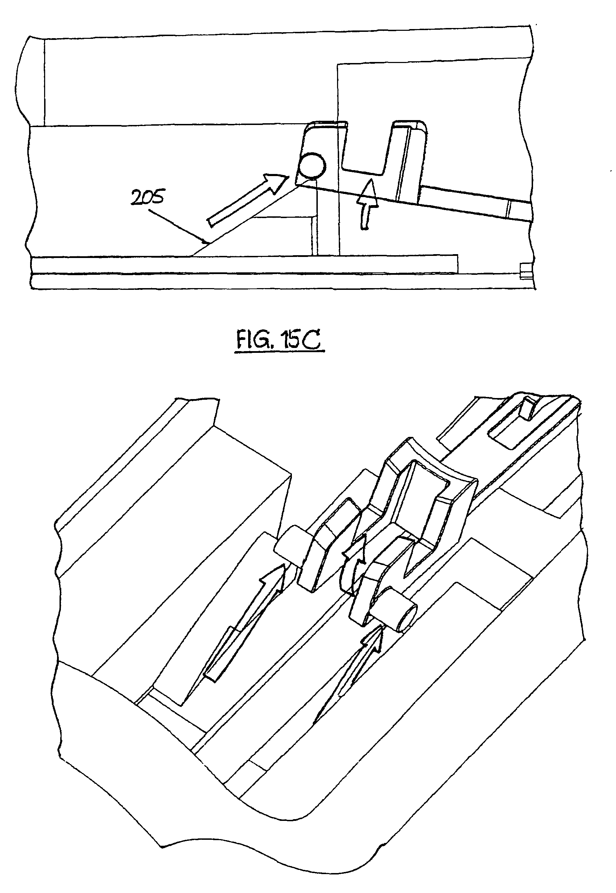

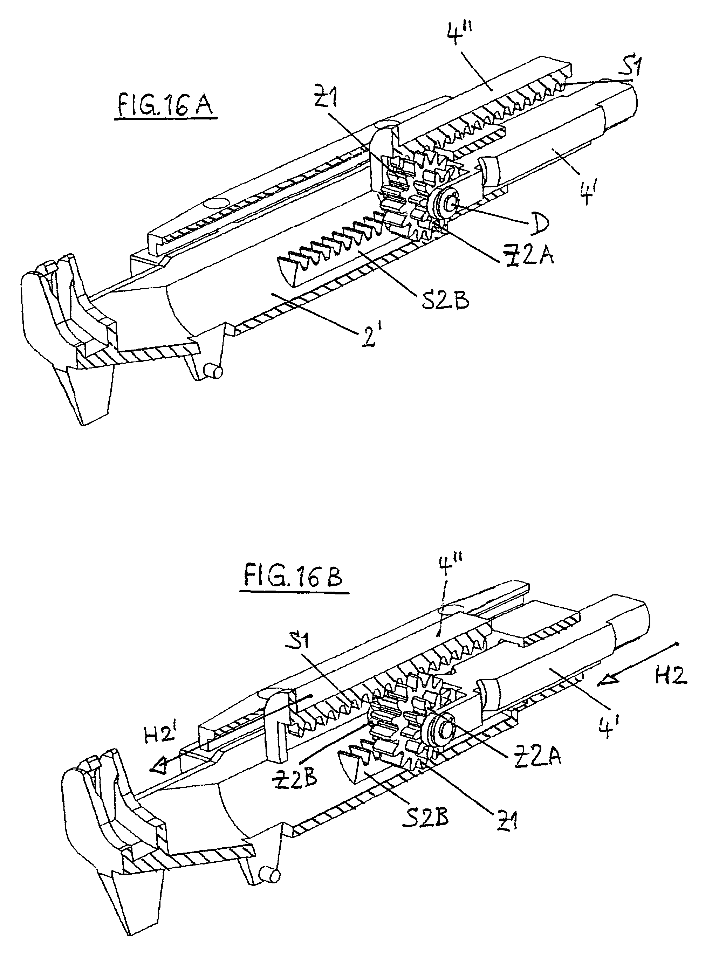

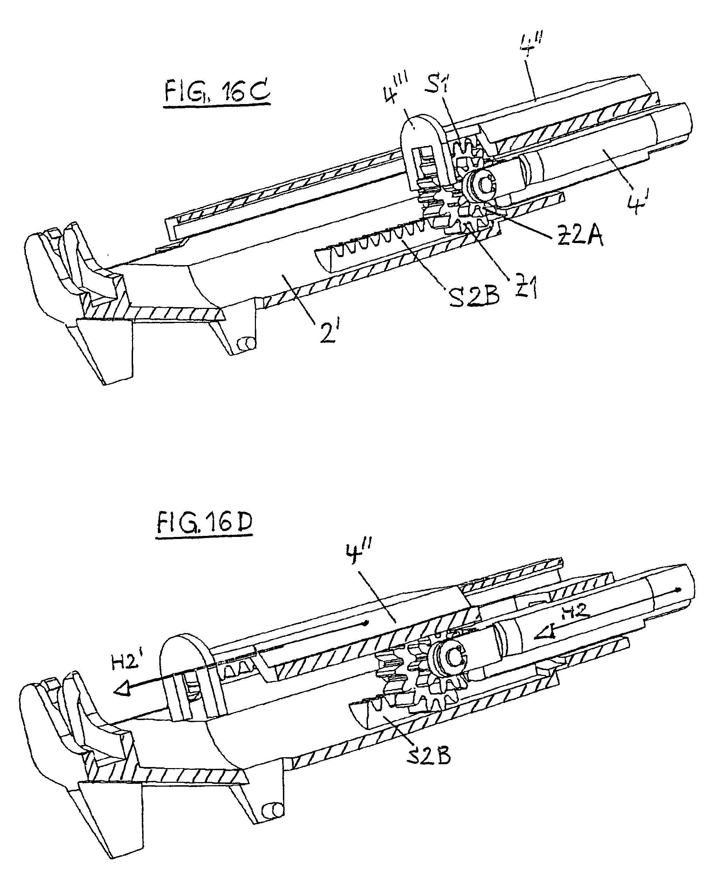

In den Figuren 16 bis 19 ist eine besonders bevorzugte Realisierung des Schlittens im Zusammenwirken mit einem Getriebe dargestellt. Durch dieses Konzept eines "Getriebeschlittens" wird es möglich, den bei dem oben beschriebenen Ausführungsbeispiel vorgegebenen zweiten Hub H2 hinsichtlich seines Wertes auf den Injektionshub einer speziellen Spritze abzustimmen, da nicht alle eingesetzten Spritzen hinsichtlich dieses Wertes normiert sind. Durch Wahl eines geeigneten Getriebes ist es also möglich, aus dem Hub H2 des Stößels 4' einen Hub H2' zu erzeugen, der geringer (Getriebeuntersetzung) oder größer (Getriebeübersetzung) ist als der Hub H2 des Stößels 4'.

Bei dem in den Figuren 16 bis 19 dargestellten Ausführungsbeispiel ist ein Getriebe mit einer Getriebeübersetzung dargestellt, d.h. der Injektionshub H2' ist größer als der Hub H2 des Stößels 4'. Es soll hierbei besonders angemerkt werden, daß die beiden Hübe H2 und H2' gleichgerichtet sind, im Gegensatz beispielweise zu Lösungen (DE 28 12 729 A1), wo zwar auch eine Getriebelösung angesprochen ist, wo aber Betätigungshub und Injektionshub gegeneinander gerichtet sind.

Die konstruktive Ausgestaltung dieses Lösungskonzepts wird nun im folgenden näher erläutert:

Der Schlitten 2' ist wannenartig ausgebildet und führt einerseits den Stößel 4', andererseits aber auch eine Schubstange 4'' parallel zueinander. Die Schubstange 4'' weist an ihrer Unterseite eine erste Zahnstange S1 auf, an ihrer Vorderseite ist eine Druckplatte 4''' angeformt, die auf den Spritzenkolben 14 einer mit ihrem Bund 12 in der Spritzenaufnahme gehaltenen Injektionsspritze 1 wirkt.

Der Stößel 4' umfaßt an seinem in Injektionsrichtung gelegenen vorderen Ende gabelartig eine Anordnung von insgesamt drei Zahnrädern, mit einem mittleren ersten Zahnrad Z1, das mit der Zahnstange S1 der Schubstange 4'' kämmt sowie beidseitig dieses zentralen ersten Zahnrads Z1 koaxial befestigten zweiten Zahnrädern Z2A,Z2B geringeren Durchmessers, die mit einer Zahnstange S2A bzw. S2B am Boden des Schlittens 2' kämmen.

Die beiden Zahnstangen S2A,S2B am Boden des wannenförmigen Schlittens 2' sind so weit beabstandet, daß der Zahnkranz des ersten Zahnrades Z1 zwischen diesen beiden Zahnstangen hineinragen kann.

Bei dem in den Figuren 16 bis 19 dargestellten bevorzugten Ausführungsbeispiel kämmt also folglich das erste Zahnrad Z1 mit der Zahnstange S1 an der Unterseite der Schubstange 4'' und die beiden seitlich angeordneten zweiten Zahnräder Z2A/Z2B kämmen mit ihrer jeweils zugeordneten zweiten Zahnstange S2A/S2B.

Bei dem in den Zeichnungen dargestellten Ausführungsbeispiel handelt es sich folglich um eine Getriebeübersetzung, d.h., der zweite Hub H2 des Stößels 4' wird in einen Hub H2' der Schubstange 4'' umgesetzt, der mehr als doppelt so groß ist wie der zweite Hub H2.

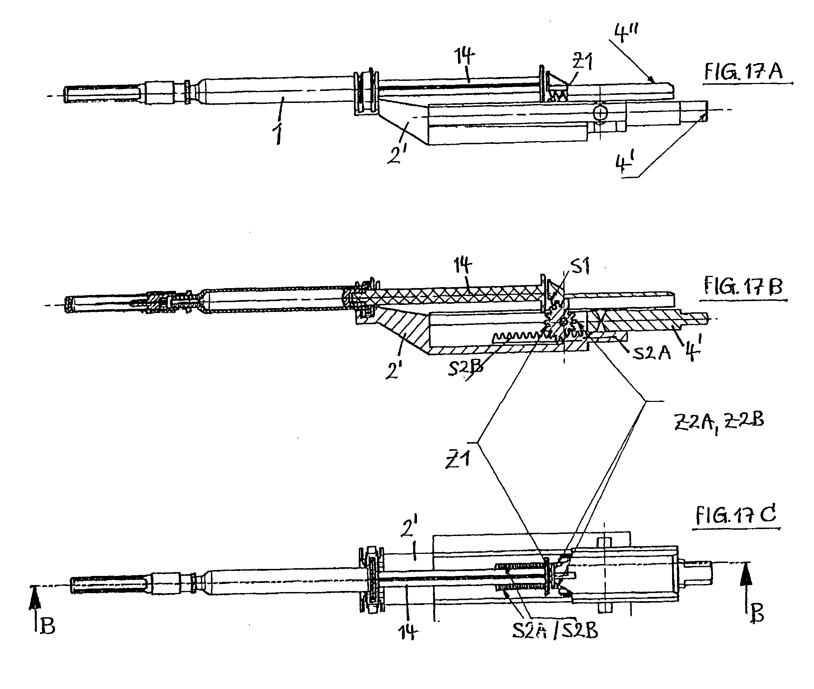

Mit dieser Vorrichtung wird also Platz geschaffen insbesondere für Injektionsspritzen 1 mit sehr langem Injektionshub und entsprechendem langen Spritzkolben 14, wie dies insbesondere aus den Figuren 17 deutlich wird.

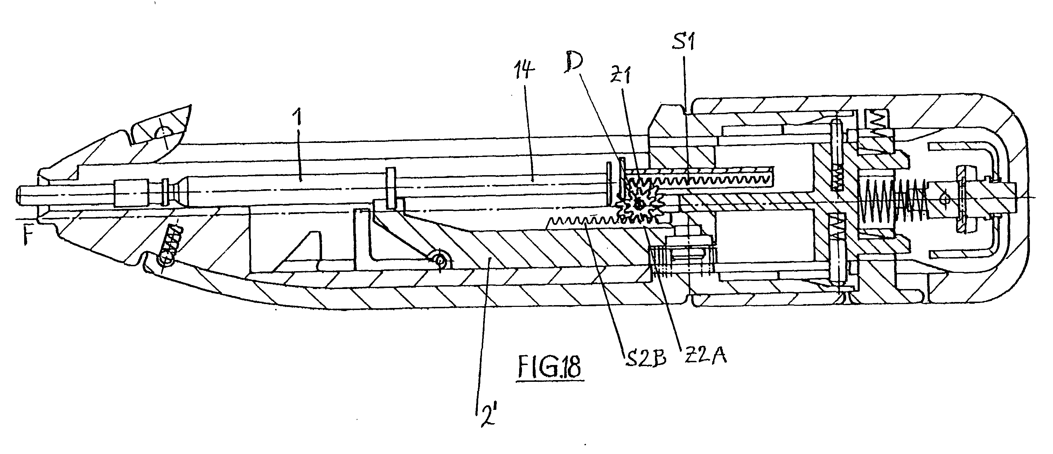

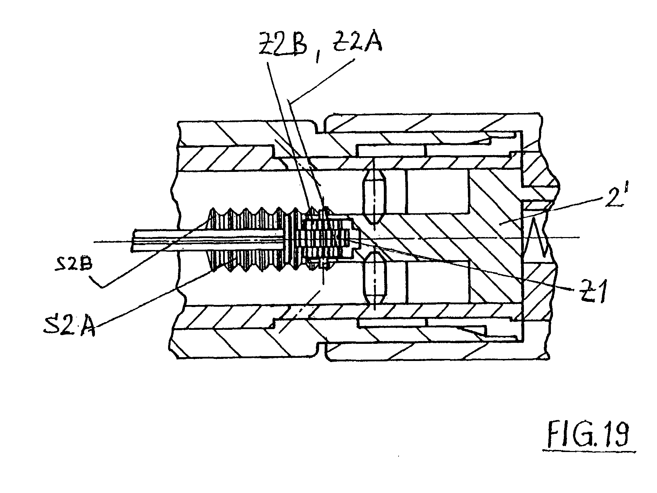

Die Gesamtkonzeption und die Wirkungsweise des Schlittens 2' als solchem im Zusammenspiel mit den anderen Bauteilen der Injektionseinrichtung bleibt hierbei voll erhalten, wie aus den Figuren 18 und 19 deutlich wird, wo anstelle des Schlittens 2 in den oben geschilderten Ausführungsbeispielen der "Getriebeschlitten" 2' eingebaut ist. Es ergibt sich hierbei lediglich insoweit eine Änderung, als daß durch die entsprechende Aufbauhöhe, die etwa dem Radius des ersten Zahnrades Z1 entspricht, eine entsprechende Abweichung der Injektionsachse von der Längsachse F des Gehäuses hervorgerufen wird, die durch entsprechende einfache Dimensionierungsvorgaben am Gehäuse berücksichtigt werden kann.

Insbesondere aus der isolierten Darstellung des "Getriebeschlittens" 2' in den Figuren 16 und 17 wird deutlich, daß eine solche Lösung nicht auf die Injektionsvorrichtung beschränkt ist, wie sie in ihren Einzelheiten eingangs beschrieben ist, sondern auch bei Injektionsvorrichtungen konventioneller Bauart mit einfachen Modifikationen angewendet bzw. dort eingesetzt werden kann. Beispielsweise läßt sich in einem solchen einfachen Fall der Schlitten 2' stationär mit einem einfachen Gehäuse verbinden und der Stößel 4' an seiner der Injektionsrichtung abgewandten Seite mit einem Betätigungsende versehen, das aus diesem Gehäuse herausragt, so daß hier der einfachste Fall einer Spritzenbetätigung ermöglicht würde, mit der gewünschten Übersetzung des Betätigungshubes in einen gleich gerichteten Injektionshub.

## Patentansprüche

1. Injektionsvorrichtung zur Betätigung einer Spritze, insbesondere einer Einmalspritze, mit in einem Gehäuse gehaltenen Antriebs- und Steuereinrichtungen, die den Injektionsvorgang sukzessiv derart ablaufen lassen, dass zunächst eine Linearverschiebung der Spritze (1) mit der Injektionsnadel zur Einführung der Injektionsnadel in die Haut und danach die Injektion der Injektionsflüssigkeit erfolgt, wobei die Antriebs- und Steuereinrichtungen eine Steuerhülse (6) beinhalten, die zwischen einer Schließ- und Funktionsposition (P1), die den Zugriff auf die Spritze (1) verhindert, und eine Auslöseeinrichtung für den Injektionsvorgang freigibt, und einer Öffnungs- und Sicherungsposition (P2), die das Einlegen und Entnehmen der Spritze (1) aus dem/in das Gehäuse (7) erlaubt, verschiebbar ist,
**dadurch gekennzeichnet, daß** die Steuerhülse (6) über einen Teil ihres Umfangs eine Ladeöffnung (L6) zum Einlegen und Entnehmen der Spritze (1) aufweist, und dass sie zwischen der Schließ- und Funktionsposition (P1) und der Öffnungs- und Sicherungsposition (P2) um einen Steuerwinkel (α) auf der Mantelfläche (M) des Gehäuses (7) verdrehbar ist.

2. Injektionsvorrichtung nach Anspruch 1, **dadurch gekennzeichnet, daß** die Steuerhülse (6) einen im wesentlichen zylindrischen Mittelabschnitt (6B) aufweist, der die Ladeöffnung (L6) aufweist.

3. Injektionsvorrichtung nach Anspruch 1, **dadurch gekennzeichnet, daß** das Gehäuse (7) einen im wesentlichen zylindrischen Mittelabschnitt (7B) aufweist, der über einen Teil seines Umfangs eine Ladeöffnung (L7) zum Einlegen und Entnehmen der Spritze (1) aufweist.

4. Injektionsvorrichtung nach Anspruch 2 und 3, **dadurch gekennzeichnet, daß** die beiden Ladeöffnungen (L6,L7) sich über einen Teil des jeweiligen Umfangs ihres zugeordneten Bauteils (6,7) erstrecken, dessen Umfangswinkel (α1,α2) größer als 180° ist, so dass in der Schließ- und Funktionsposition (P1) die Spritze (1) vollständig vom Gehäuse (7) und der Steuerhülse (6) umschlossen ist, und in der um den Steuerwinkel (α) verdrehten Öffnungs- und Sicherungsposition (P2) der Steuerhülse (6) die beiden Ladeöffnung (L6,L7) zumindest teilweise deckungsgleich sind.

5. Injektionsvorrichtung nach Anspruch 1, **dadurch gekennzeichnet, daß** ein erster Hub (H1) eines Schlittens (2), in dem die Spritze (1) gehalten ist, zur Einfahrung der Injektionsnadel (13) innerhalb des Gehäuses (7) zwischen einer Arretierposition (A1) und einer Vorschubposition (V1) vorgesehen ist.

6. Injektionsvorrichtung nach Anspruch 1 und 5, **dadurch gekennzeichnet, daß** ein zweiter Hub (H2) eines Stößels (4) relativ zum Schlitten (2) zur Injektion der Injektionsflüssigkeit Innerhalb des Gehäuses (7) zwischen einer Arretierposition (A2) und einer Vorschubposition (V2) vorgesehen ist.

7. Injektionsvorrichtung nach Anspruch 5 und 6, **dadurch gekennzeichnet, daß** die Relativpositionen von Stößel (4) und Schiltten (2) durch ein Kopplungselement (8) so definiert sind, daß nach Betätigung der Auslöseeinrichtung der zweite Hub (H2) des Stößels (4) unmittelbar auf den ersten Hub (H1) des Schlittens (2) folgt und die beiden Hübe (H1,H2) sich addieren.

8. Injektionsvorrichtung nach Anspruch 7, **dadurch gekennzeichnet, daß** das Kopplungselement (8) während des ersten Hubs (H1) des Schlittens (2) diesen und den Stößel (4) über ein erstes Rastelement (81) im Stößel (4) miteinander koppelt und nach dem ersten Hub (H1) mittels eines zweiten Rastelements (72) im Gehäuse (7) die Kopplung löst, so daß dann der Stößel (4) alleine den zweiten Hub (H2) durchführt.

9. Injektionsvorrichtung nach Anspruch 1, **dadurch gekennzeichnet, daß** Steuerhülse (6), Gehäuse (7), Schlitten (2) und Stößel (4) zumindest abschnittweise als koaxial zueinander liegende Zylinder- oder Hohlzylinderabschnitte ausgebildet sind.

10. Injektionsvorrichtung nach Anspruch 1 und 9, **dadurch gekennzeichnet, daß** das Gehäuse (7) in einem rückwärtigen, zylindrischen Handhabungsabschnitt (7A) eine Ringnut (75) aufweist, in der ein zylindrischer Steuerabschnitt (6A) der Steuerhülse (6) verdrehbar geführt ist.

11. Injektionsvorrichtung nach Anspruch 1,9 und 10, **dadurch gekennzeichnet, daß** die Steuerhülse (6) einen vorderen, verjüngten Führungsabschnitt (6C) aufweist, der auf dem Gehäuse (7) drehbar gehalten ist.

12. Injektionsvorrichtung nach Anspruch 1, **dadurch gekennzeichnet, daß** die Schließposition (P1) und die Öffnungsposition (P2) durch ein zwischen Gehäuse (7) und Steuerhülse (6) wirkendes Rastelement (77) und durch um den Steuerwinkel (α) gegeneinander versetzt wirkende Vertiefungen (77A,77B) im Gehäuse (7) gesichert wird.

13. Injektionsvorrichtung nach Anspruch 1 und 9, **dadurch gekennzeichnet, daß** der Schlitten (2) über einen im Gehäuse (7) geführten ersten Abschnitt (2A) als Hohlzylinder ausgebildet ist, in dem der Stößel (4) mittig axial verschiebbar gelagert ist.

14. Injektionsvorrichtung nach Anspruch 13, **dadurch gekennzeichnet, daß** der Schlitten (2) in einem im Gehäuse (7) geführten zweiten Abschnitt (2B) einen U-förmigen Querschnitt aufweist, sowie eine nutförmige oder schlitzartige Halterung (21) zur Positionierung des in dieser Halterung (21) derart gehaltenen Spritzenbundes (12), daß die Stirnseite des Spritzenkolbens vom Stößel (4) in Injektionsrichtung verschiebbar ist.

15. Injektionsvorrichtung nach Anspruch 9, **dadurch gekennzeichnet, daß** der Stößel (4) an seinem zum Handhabungsabschnitt (7A) des Gehäuses (7) zeigenden Ende einen im Gehäuse (7) geführten Führungsabschnitt (4A) aufweist, der den gleichen Außendurchmesser aufweist wie der erste Abschnitt (2A) des Schlittens (2).

16. Injektionsvorrichtung nach Anspruch 15, **dadurch gekennzeichnet, daß** zwischen der rückwärtigen Stirnseite des Führungsabschnitts (4A) des Stößels (4) und der Gehäuserückwand (74) eine gemeinsame Antriebseinrichtung zur Erzeugung der beiden Hübe (H1,H2) durch den Schlitten (2) und den Stößel (4) untergebracht ist.

17. Injektionsvorrichtung nach Anspruch 1, 6 und 15, **dadurch gekennzeichnet, daß** an der Rückseite des Führungsabschnitts (4A) des Stößels (4) mindestens eine Sicherungsklinke (41) angeformt ist, die in der Öffnungs- und Sicherungsposition (P2) mit mindestens einer quer zur Längsachse (F) verschiebbaren Steuerklinke (53,54) der Auslöseeinrichtung in lösbarem Eingriff steht und den Stößel (4) gegen die Vorspannung der Antriebseinrichtung in seiner Arretierposition (A1) hält.

18. Injektionsvorrichtung nach Anspruch 17, **dadurch gekennzeichnet, daß** die Auslöseeinrichtung durch das Gehäuse (7) nach außen geführt ist und dort eine Betätigungstaste (5) zur Freigabe der Sicherungsklinke (41) und damit der beiden Hübe (H1,H2) bildet.

19. Injektionsvorrichtung nach Anspruch 18, **dadurch gekennzeichnet, daß** die Verschiebung der Betätigungstaste (5) nur möglich ist, wenn die Steuerhülse (6) sich in der Schließ- und Funktionsposition (P1) befindet.

20. Injektionsvorrichtung nach Anspruch 10 und 18, **dadurch gekennzeichnet, daß** der zylindrische Steuerabschnitt (6A) der Steuerhülse (6) eine randseitige Ausnehmung (6A1) aufweist, durch die in der Schließ- und Funktionsposition (P1) eine Nase (5A) der Betätigungstaste (5) passieren kann.

21. Injektionsvorrichtung nach Anspruch 15, **dadurch gekennzeichnet, daß** im Führungsabschnitt (4A) des Stößels (4) Steuerelemente gehalten sind, die mit auf der inneren Mantelfläche (Ml) des zylindrischen Steuerabschnitts (6A) der Steuerhülse (6) gebildeten Profilen zusammenwirken, die mindestens eine Steuerkurve bilden.

22. Injektionsvorrichtung nach Anspruch 21, **dadurch gekennzeichnet, daß** die Steuerelemente aus mindestens einem Hubbolzen (42,43) besteht/bestehen, der/die Steuerkurvenabschnitte (64,67;65,66;68,69) derart beaufschlagt/beaufschlagen, daß durch eine Verdrehung der Steuerhülse (6) von der Schließ- und Funktionsposition (P1) nach Durchfahrung der beiden Hübe (H1,H2) um den Funktionswinkel in die Öffnungs- und Sicherungsposition (P2) das hierbei aufgebrachte Drehmoment über die Steuerkurve(n)abschnitte (64,67;65,66;68,69) in eine lineare Rückstellkraft gegen die Spannkraft der Spiralfeder (3) umgesetzt wird, wodurch der Stößel (4) und der Schlitten (2) im Gehäuse (7) einen Gegenhub ( H) ausführen, bis in den Arretierpositionen (A1,A2) die Sicherungsklinken (41) des Stößels (4) wieder in Eingriff mit den Steuerklinken (53,54) der Auslöseeinrichtung (5) kommen.

23. Injektionsvorrichtung nach Anspruch 21 und 22, **dadurch gekennzeichnet, daß** zwei unterschiedlich dicke, in Richtung zur Mantelfläche (Ml) federbelastete Hubholzen (42,43) vorgesehen sind, die von zwei in der inneren Mantelfläche (Ml) höhenversetzt angeordneten Steuerkurven (64,67 und 65,66) derart geführt werden, daß in jeder Axialposition des Stößels (4) die Umsetzung des Drehmoments der Steuerhülse (6) über zumindest einen der Hubbolzen (42,43) erfolgt.

24. Injektionsvorrichtung nach Anspruch 1, **dadurch gekennzeichnet, daß** die Steuerhülse (6) in der Schließ- und Funktionsposition (P1) den Blick auf die Spritze (1) erlaubt.

25. Injektionsvorrichtung nach Anspruch 3 und 22, **dadurch gekennzeichnet, daß** gegenüber der Ladeöffnung (L7) des Gehäuses (7) eine Auswurfeinrichtung vorgesehen ist, die nach erfolgter Injektion bei der Freigabe der Ladeöffnung (L7) durch Drehung der Steuerhülse (6) in die Öffnungs- und Sicherungsposition (P2) die Spritze (1) in Richtung zu den Ladeöffnungen (L6, L7) hin anhebt.

26. Injektionsvorrichtung nach Anspruch 1, **dadurch gekennzeichnet, daß** eine Signaleinrichtung vorgesehen ist, die nach erfolgter Injektion am Ende des zweiten Hubes (H2) betätigt wird und mittels mechanisch wirkender Bauelemente ein akustisches Signal erzeugt.

27. Injektionsvorrichtung nach Anspruch 6, **dadurch gekennzeichnet, daß** der Stößel (4') über ein Lineargetriebe mit einer Schubstange (4 " ) gekoppelt ist, die den Spritzenkolben (14) betätigt.

28. Injektionsvorrichtung nach Anspruch 27, **dadurch gekennzeichnet, daß** die Übersetzung des Getriebes so gewählt ist, daß der zweite Hub (H2) des Stößels (4') in einen gleichgerichteten Hub der Schubstange (4" ) umgesetzt wird, der dem Injektionshub des Spritzenkolbens (14) entspricht.

29. Injektionsvorrichtung nach Anspruch 28, **dadurch gekennzeichnet, daß** die Schubstange (4 " ) parallel zum Stößel (4') vom Schlitten (2') geführt ist.

30. Injektionsvorrichtung nach Anspruch 27 bis 29, **dadurch gekennzeichnet, daß** im vorderen Abschnitt des Stößels (4') mindestens zwei Zahnräder (Z1,Z2) unterschiedlichen Durchmessers koaxial aneinander befestigt sind, von denen das erste (Z1) mit einer ersten Zahnstange (S1) an der Schubstange (4 ") und das zweite (Z2) mit einer zweiten Zahnstange (S2) am Schlitten (2') kämmt.

31. Injektionsvorrichtung nach Anspruch 27, **dadurch gekennzeichnet, daß** die Längsachse der Spritze (1) parallel zur Längsachse (F) des Gehäuses (7) liegt.

32. Injektionsvorrichtung nach Anspruch 27 bis 29, **dadurch gekennzeichnet, daß** im vorderen Abschnitt des Stößels ein Zahnrad gelagert ist, das mit einer ersten Zahnstange an der Schubstange und mit einer zweiten Zahnstange am Schlitten kämmt, so daß der Injektionshub doppelt so groß ist wie der Hub des Stößels.

## Claims

1. Injection device for operating a syringe, more especially a disposable syringe, said device having driving and controlling means which are retained in a housing and permit the injection operation to occur successively in such a manner that initially the syringe (1), with the injection needle, is linearly displaced to introduce the injection needle into the skin, and then the injection fluid is injected, the driving and controlling means including a control sleeve (6), which is displaceable between a closed and operational position (P1), which prevents access to the syringe (1) and activates a release mechanism for the injection operation, and an open and safety position (P2), which permits the syringe (1) to be inserted into and removed from the housing (7), **characterised in that** the control sleeve (6) has, over some of its circumference, a loading aperture (L6) for the insertion and removal of the syringe (1), and **in that** said sleeve is rotatable through a control angle (α) on the surface (M) of the housing (7) between the closed and operational position (P1) and the open and safety position (P2).

2. Injection device according to claim 1, **characterised in that** the control sleeve (6) has a substantially cylindrical central portion (6B), which has the loading aperture (L6).

3. Injection device according to claim 1, **characterised in that** the housing (7) has a substantially cylindrical central portion (7B), which has a loading aperture (L7) over some of its circumference for the insertion and removal of the syringe (1).

4. Injection device according to claims 2 and 3, **characterised in that** the two loading apertures (L6, L7) extend over some of the pertinent circumference of their associated structural member (6, 7), the circumferential angle (α1, α2) of which component is greater than 180° so that, in the closed and operational position (P1), the syringe (1) is completely surrounded by the housing (7) and the control sleeve (6), and the two loading apertures (L6, L7) are at least partially identical in area in the open and safety position (P2) of the control sleeve (6) rotated through the control angle (α).

5. Injection device according to claim 1, **characterised in that** a first stroke (H1) of a slide (2), in which the syringe (1) is retained, is provided in order to introduce the injection needle (13) inside the housing (7) between a locked position (A1) and a feed position (V1).

6. Injection device according to claims 1 and 5, **characterised in that** a second stroke (H2) of a plunger (4) is provided relative to the slide (2) for the injection of the injection fluid inside the housing (7) between a locked position (A2) and a feed position (V2).

7. Injection device according to claims 5 and 6, **characterised in that** the relative positions of plunger (4) and slide (2) are defined by a coupling member (8) so that, after actuation of the release mechanism, the second stroke (H2) of the plunger (4) follows directly on the first stroke (H1) of the slide (2), and the two strokes (H1, H2) are added together.

8. Injection device according to claim 7, **characterised in that** the coupling member (8) joins together the slide (2) and the plunger (4) via a first locking member (81) in the plunger (4) during the first stroke (H1) of said slide and, after the first stroke (H1), releases the connection by means of a second locking member (72) in the housing (7), so that the plunger (4) then accomplishes the second stroke (H2) on its own.

9. Injection device according to claim 1, **characterised in that** control sleeve (6), housing (7), slide (2) and plunger (4) are, at least in sections, in the form of cylinder or hollow-cylinder portions which lie coaxially relative to one another.

10. Injection device according to claims 1 and 9, **characterised in that** the housing (7) has, in a rear, cylindrical operating portion (7A), an annular groove (75) in which a cylindrical control portion (6A) of the control sleeve (6) is rotatably guided.

11. Injection device according to claims 1, 9 and 10, **characterised in that** the control sleeve (6) has a front, tapering guide portion (6C), which is rotatably retained on the housing (7).

12. Injection device according to claim 1, **characterised in that** the closed position (P1) and the open position (P2) are secured by a locking member (77), which acts between housing (7) and control sleeve (6), and by indentations (77A, 77B) in the housing (7), which indentations act so as to be offset from one another by the control angle (α).

13. Injection device according to claims 1 and 9, **characterised in that** the slide (2) is in the form of a hollow cylinder over a first portion (2A), which is guided in the housing (7), the plunger (4) being centrally axially displaceably mounted in said cylinder.

14. Injection device according to claim 13, **characterised in that** the slide (2) has a U-shaped cross-section in a second portion (2B), which is guided in the housing (7), and said slide also has a groove-like or slot-like holder (21) for positioning the syringe collar (12), which is retained in this holder (21) in such a manner that the end face of the syringe piston is displaceable in the direction of injection by the plunger (4).

15. Injection device according to claim 9, **characterised in that** the plunger (4) has, at its end pointing towards the operating portion (7A) of the housing (7), a guide portion (4A), which is guided in the housing (7) and has the same external diameter as the first portion (2A) of the slide (2).

16. Injection device according to claim 15, **characterised in that** a common driving means is accommodated between the rear end face of the guide portion (4A) of the plunger (4) and the rear housing wall (74) to produce the two strokes (H1, H2) by the slide (2) and the plunger (4).

17. Injection device according to claims 1, 6 and 15, **characterised in that** at least one safety catch (41) is provided on the rear side of the guide portion (4A) of the plunger (4), said safety catch being in detachable engagement with at least one control pawl (53, 54) of the release mechanism in the open and safety position (P2) and retaining the plunger (4) in its locked position (A1) in opposition to the initial tension of the driving means, which control pawl is displaceable transversely relative to the longitudinal axis (F).

18. Injection device according to claim 17, **characterised in that** the release mechanism is guided outwardly through the housing (7) and forms there an actuating button (5) for activating the safety catch (41) and, hence, the two strokes (H1, H2).

19. Injection device according to claim 18, **characterised in that** the displacement of the actuating button (5) is only possible when the control sleeve (6) is in the closed and operational position (P1).

20. Injection device according to claims 10 and 18, **characterised in that** the cylindrical control portion (6A) of the control sleeve (6) has a recess (6A1) on the edge side, through which recess a projection (5A) of the actuating button (5) can pass in the closed and operational position (P1).

21. Injection device according to claim 15, **characterised in that**, in the guide portion (4A) of the plunger (4), control members are retained which co-operate with profiles, which are formed on the internal surface (Ml) of the cylindrical control portion (6A) of the control sleeve (6) and form at least one cam.

22. Injection device according to claim 21, **characterised in that** the control members comprise at least one lifting pin (42, 43), which acts upon cam portions (64, 67; 65, 66; 68, 69) in such a manner that, by a rotational movement of the control sleeve (6) from the closed and operational position (P1), after the two strokes (H1, H2) have passed through the operational angle, into the open and safety position (P2), the rotary moment applied hereby is converted, via the cam portions (64, 67; 65, 66; 68, 69), into a linear restoring force in opposition to the tensioning force of the helical spring (3), whereby the plunger (4) and the slide (2) in the housing (7) exert a counter-stroke (H) until, in the locked positions (A1, A2), the safety catches (41) of the plunger (4) come into engagement again with the control pawls (53, 54) of the release mechanism (5).

23. Injection device according to claims 21 and 22, **characterised in that** two variably thick lifting pins (42, 43) are provided, which are spring-loaded in the direction of the surface (Ml) and are guided by cams (64, 67 and 65, 66), disposed in the internal surface (Ml) in a vertically offset manner, in such a manner that, in each axial position of the plunger (4), the moment of rotation of the control sleeve (6) is converted via at least one of the lifting pins (42, 43).

24. Injection device according to claim 1, **characterised in that** the control sleeve (6) permits the syringe (1) to be viewed in the closed and operational position (P1).

25. Injection device according to claims 3 and 22, **characterised in that** an ejection means is provided opposite the loading aperture (L7) of the housing (7), which means raises the syringe (1) towards the loading apertures (L6, L7), after injection has taken place, with the activation of the loading aperture (L7) by rotating the control sleeve (6) into the open and safety position (P2).

26. Injection device according to claim 1, **characterised in that** a signalling means is provided which, after injection has taken place, is actuated at the end of the second stroke (H2) and generates an acoustic signal by means of mechanically acting structural elements.

27. Injection device according to claim 6, **characterised in that** the plunger (4') is connected, via a linear transmission, to a push-rod (4") which actuates the syringe piston (14).

28. Injection device according to claim 27, **characterised in that** the speed change of the transmission is so selected that the second stroke (H2) of the plunger (4') is converted into an identically orientated stroke of the push-rod (4"), which stroke corresponds to the injection stroke of the syringe piston (14).

29. Injection device according to claim 28, **characterised in that** the push-rod (4") is guided parallel to the plunger (4') by the slide (2').

30. Injection device according to claims 27 to 29, **characterised in that**, in the front portion of the plunger (4'), at least two toothed wheels (Z1, Z2) of variable diameter are mounted coaxially adjacent each other, of which the first toothed wheel (Z1) meshes with a first toothed rod (S1) on the push-rod (4"), and the second toothed wheel (Z2) meshes with a second toothed rod (S2) on the slide (2').

31. Injection device according to claim 27, **characterised in that** the longitudinal axis of the syringe (1) lies parallel to the longitudinal axis (F) of the housing (7).

32. Injection device according to claims 27 to 29, **characterised in that**, in the front portion of the plunger, a toothed wheel is mounted, which meshes with a first toothed rod on the push-rod and with a second toothed rod on the slide, so that the injection stroke is twice as large as the stroke of the plunger.

## Revendications

1. Dispositif d'injection pour l'actionnement d'une seringue, en particulier une seringue à usage unique, comprenant des dispositifs de commande et d'entraînement maintenus dans un boîtier et qui assurent un déroulement successif de l'opération d'injection tel, qu'un déplacement linéaire de la seringue (1) avec l'aiguille d'injection est d'abord assuré pour l'introduction de l'aiguille dans la peau, et que le liquide est ensuite injecté, les dispositifs de commande et d'entraînement renfermant une douille de commande (6) déplaçable entre une position de fermeture et de fonctionnement (P1), qui empêche l'accès à la seringue (1) et libère un dispositif de déclenchement pour l'opération d'injection, et une position d'ouverture et de blocage (P2), qui permet l'insertion et le retrait de la seringue (1) dans/du boîtier (7), **caractérisé en ce que** la douille de commande (6) présente sur une partie de son pourtour une ouverture de chargement (L6) pour l'insertion et le retrait de la seringue (1), et **en ce qu'**elle peut être tournée entre la position de fermeture et de fonctionnement (P1) et la position d'ouverture et de blocage (P2), d'un angle de commande (α), sur la surface d'enveloppe (M) du boîtier (7).

2. Dispositif d'injection suivant la revendication 1, **caractérisé en ce que** la douille de commande (6) présente une section centrale (6B) essentiellement cylindrique, qui comporte l'ouverture de chargement (L6).

3. Dispositif d'injection suivant la revendication 1, **caractérisé en ce que** le boîtier (7) présente une section centrale (7B) essentiellement cylindrique, qui comporte sur une partie de son pourtour une ouverture de chargement (L7) pour l'insertion et le retrait de la seringue (1).

4. Dispositif d'injection suivant les revendications 2 et 3, **caractérisé en ce que** les deux ouvertures de chargement (L6, L7) s'étendent sur une partie du pourtour respectif de leur composant associé (6, 7) dont l'angle inscrit (α1, α2) est supérieur à 180°, de sorte que la seringue (1) est totalement enveloppée par le boîtier (7) et par la douille de commande (6) dans la position de fermeture et de fonctionnement (P1), et que les deux ouvertures de chargement (L6, L7) sont coïncidentes, au moins en partie, dans la position d'ouverture et de blocage (P2), tournée de l'angle de commande (α), de la douille de commande (6).

5. Dispositif d'injection suivant la revendication 1, **caractérisé en ce qu'**une première course (H1) d'un coulisseau (2), dans lequel est maintenue la seringue (1), est prévue à l'intérieur du boîtier (7) entre une position d'arrêt (A1) et une position d'avance (V1), pour la rentrée de l'aiguille d'injection (13).

6. Dispositif d'injection suivant les revendications 1 et 5, **caractérisé en ce qu'**une seconde course (H2) d'un poussoir (4) par rapport au coulisseau (2) est prévue à l'intérieur du boîtier (7) entre une position d'arrêt (A2) et une position d'avance (V2), pour l'injection du liquide à injecter.

7. Dispositif d'injection suivant les revendications 5 et 6, **caractérisé en ce que** les positions relatives du poussoir (4) et du coulisseau (2) sont définies par un élément d'accouplement (8) de sorte que, après l'actionnement du dispositif de déclenchement, la seconde course (H2) du poussoir (4) suit immédiatement la première course (H1) du coulisseau (2) et que les deux courses (H1, H2) s'additionnent.

8. Dispositif d'injection suivant la revendication 7, **caractérisé en ce que** l'élément d'accouplement (8), pendant la première course (H1) du coulisseau (2), accouple mutuellement ce dernier et le poussoir (4) par l'intermédiaire d'un premier élément d'encliquetage (81) prévu dans le poussoir (4), et débloque l'accouplement après la première course (H1) au moyen d'un second élément d'encliquetage (72) prévu dans le boîtier (7), de sorte que le poussoir (4) exécute alors seul la seconde course (H2).

9. Dispositif d'injection suivant la revendication 1, **caractérisé en ce que** la douille de commande (6), le boîtier (7), le coulisseau (2) et le poussoir (4) sont réalisés, au moins par sections, sous forme de sections cylindriques ou de sections cylindriques creuses coaxiales entre elles.

10. Dispositif d'injection suivant les revendications 1 et 9, **caractérisé en ce que** le boîtier (7) présente dans une section de manipulation (7A) arrière, cylindrique, une rainure annulaire (75) dans laquelle est guidée en rotation une section de commande cylindrique (6A) de la douille de commande (6).

11. Dispositif d'injection suivant les revendications 1, 9 et 10, **caractérisé en ce que** la douille de commande (6) présente une section de guidage avant (6C), rétrécie, maintenue en rotation sur le boîtier (7).

12. Dispositif d'injection suivant la revendication 1, **caractérisé en ce que** la position de fermeture (P1) et la position d'ouverture (P2) sont bloquées par un élément d'encliquetage (77) agissant entre le boîtier (7) et la douille de commande (6), et par des cavités (77A, 77B) dans le boîtier (7) agissant en déport mutuel de l'angle de commande (α).

13. Dispositif d'injection suivant les revendications 1 et 9, **caractérisé en ce que** le coulisseau (2) est réalisé sous forme de cylindre creux sur une première section (2A) guidée dans le boîtier (7), cylindre au centre duquel le poussoir (4) est monté avec une possibilité de déplacement dans le sens axial.

14. Dispositif d'injection suivant la revendication 13, **caractérisé en ce que** le coulisseau (2) présente une section transversale en U dans une seconde section (2B) guidée dans le boîtier (7), ainsi qu'une fixation (21) en forme de rainure ou de fente pour le positionnement de la collerette de seringue (12), maintenue dans cette fixation (21) de sorte que le côté frontal du piston de seringue peut être déplacé par le poussoir (4) dans le sens d'injection.

15. Dispositif d'injection suivant la revendication 9, **caractérisé en ce que** le poussoir (4) présente, sur son extrémité tournée vers la section de manipulation (7A) du boîtier (7), une section de guidage (4A) guidée dans le boîtier (7) et dotée du même diamètre extérieur que la première section (2A) du coulisseau (2).

16. Dispositif d'injection suivant la revendication 15, **caractérisé en ce qu'**un dispositif d'entraînement commun pour la production des deux courses (H1, H2) par le coulisseau (2) et le poussoir (4) est logé entre le côté frontal arrière de la section de guidage (4A) du poussoir (4) et la paroi arrière (74) du boîtier.

17. Dispositif d'injection suivant les revendications 1, 6 et 15, **caractérisé en ce qu'**au moins un cliquet d'arrêt (41) est conformé sur le côté arrière de la section de guidage (4A) du poussoir (4), ce cliquet étant en prise amovible, dans la position d'ouverture et de blocage (P2), avec au moins un cliquet de commande (53, 54), mobile transversalement à l'axe longitudinal (F), du dispositif de déclenchement, et maintenant le poussoir (4) dans sa position d'arrêt (A2) contre la précontrainte du dispositif d'entraînement.

18. Dispositif d'injection suivant la revendication 17, **caractérisé en ce que** le dispositif de déclenchement est guidé vers l'extérieur au travers du boîtier (7), et forme à cet endroit une touche d'actionnement (5) pour débloquer le cliquet d'arrêt (41) et ainsi les deux courses (H1, H2).

19. Dispositif d'injection suivant la revendication 18, **caractérisé en ce que** le déplacement de la touche d'actionnement (5) n'est possible que lorsque la douille de commande (6) se situe dans la position de fermeture et de fonctionnement (P1).

20. Dispositif d'injection suivant les revendications 10 et 18, **caractérisé en ce que** la section de commande cylindrique (6A) de la douille de commande (6) présente un creux (6A1) côté bordure, au travers duquel peut passer un ergot (5A) de la touche d'actionnement (5) dans la position de fermeture et de fonctionnement (P1).

21. Dispositif d'injection suivant la revendication 15, **caractérisé en ce que** des éléments de commande sont maintenus dans la section de guidage (4A) du poussoir (4), ces éléments concourant avec des profilés formés sur la surface d'enveloppe intérieure (MI) de la section de commande cylindrique (6A) de la douille de commande (6), qui forment au moins une came de commande.

22. Dispositif d'injection suivant la revendication 21, **caractérisé en ce que** les éléments de commande se composent d'au moins un axe de levage (42, 43) qui sollicite des sections de cames de commande (64, 67 ; 65, 66 ; 68, 69) de sorte que, par une rotation de la douille de commande (6) de la position de fermeture et de fonctionnement (P1) après le passage des deux courses (H1, H2), de l'angle fonctionnel, dans la position d'ouverture et de blocage (P2), le couple alors appliqué est converti par l'intermédiaire des sections de came(s) de commande (64, 67 ; 65, 66 ; 68, 69) en une force de rappel linéaire contre la force de tension du ressort en spirale (3), le poussoir (4) et le coulisseau (2) exécutant de ce fait une contre-course (ΔH) dans le boîtier (7), jusqu'à ce que les cliquets d'arrêt (41) du poussoir (4) entrent de nouveau en prise avec les cliquets de commande (53, 54) du dispositif de déclenchement (5) dans les positions d'arrêt (A1, A2).

23. Dispositif d'injection suivant les revendications 21 et 22, **caractérisé en ce qu'**il est prévu deux axes de levage (42, 43) d'épaisseur différente, chargés par ressort en direction de la surface d'enveloppe (MI), qui sont guidés par deux cames de commande (64, 67 et 65, 66) déportées en hauteur dans la surface d'enveloppe intérieure (MI) de sorte que, dans chaque position axiale du poussoir (4), la conversion du couple de la douille de commande (6) est assurée par l'intermédiaire d'au moins l'un des axes de levage (42, 43).

24. Dispositif d'injection suivant la revendication 1, **caractérisé en ce que** la douille de commande (6) permet de voir la seringue (1) dans la position de fermeture et de fonctionnement (P1).

25. Dispositif d'injection suivant les revendications 3 et 22, **caractérisé en ce qu'**un dispositif d'éjection est prévu en vis-à-vis de l'ouverture de chargement (L7) du boîtier (7), ce dispositif, une fois l'injection assurée, soulevant la seringue (1) en direction des ouvertures de chargement (L6, L7) lors du dégagement de l'ouverture de chargement (L7) par rotation de la douille de commande (6) dans la position d'ouverture et de blocage (P2).

26. Dispositif d'injection suivant la revendication 1, **caractérisé en ce qu'**il est prévu un dispositif de signalisation, qui est actionné une fois l'injection assurée à la fin de la seconde course (H2) et génère un signal acoustique au moyen de composants agissant mécaniquement.

27. Dispositif d'injection suivant la revendication 6, **caractérisé en ce que** le poussoir (4') est couplé par l'intermédiaire d'un engrenage linéaire à une tige de poussée (4''), qui actionne le piston de seringue (14).

28. Dispositif d'injection suivant la revendication 27, **caractérisé en ce que** le rapport de l'engrenage est choisi de sorte que la seconde course (H2) du poussoir (4') est transformée en une course de même sens de la tige de poussée (4''), qui correspond à la course d'injection du piston de seringue (14).

29. Dispositif d'injection suivant la revendication 28, **caractérisé en ce que** la tige de poussée (4'') est guidée parallèlement au poussoir (4') par le coulisseau (2').

30. Dispositif d'injection suivant les revendications 27 à 29, **caractérisé en ce qu'**au moins deux roues dentées (Z1, Z2) de diamètre différent sont fixées entre elles de façon coaxiale dans la section avant du poussoir (4'), la première roue (21) engrenant avec une première crémaillère (S1) sur la tige de poussée (4'') et la seconde (Z2) engrenant avec une seconde crémaillère (S2) sur le coulisseau (2').

31. Dispositif d'injection suivant la revendication 27, **caractérisé en ce que** l'axe longitudinal de la seringue (1) est parallèle à l'axe longitudinal (F) du boîtier (7).

32. Dispositif d'injection suivant les revendications 27 à 29, **caractérisé en ce qu'**une roue dentée est montée dans la section avant du poussoir, cette roue engrenant avec une première crémaillère sur la tige de poussée et avec une seconde crémaillère sur le coulisseau, de sorte que la course d'injection est deux fois plus élevée que la course du poussoir.
